# EUROPEAN PATENT APPLICATION

(11) **EP 2 085 473 A1**
(43) Date of publication of application: **05.08.2009**
(21) Application number: 07829955.9
(22) Date of filing: 10.10.2007
(51) Int. Cl.: C12N 15/00

(54) **AUXILIARY REAGENT FOR GENE TRANSFER**

(30) Priority: 10.10.2006 JP 2006276322
(71) Applicant: Nihon University, Tokyo 102-8275 (JP)
(72) Inventor: HIDAI, Chiaki, Tokyo 102-8275 (JP); KITANO, Hisataka, Tokyo 102-8275 (JP)
(74) Representative: Denison, Christopher Marcus
(86) International application number: PCT/JP2007/070221
(87) International publication number: WO 2008/044794

(57) **Abstract**

The present invention provides a means for introducing a gene of interest into a host cell with a higher degree of efficiency regardless of the gene transfer method employed and the type of the host cell. The auxiliary reagent for gene transfer of the present invention is **characterized in that** it comprises a peptide comprising a specific amino acid sequence represented by formula (I), a mutant or derivative thereof, or a salt of the peptide, the mutant or the derivative.

## Description

### TECHNICAL FIELD

The present invention relates to an auxiliary reagent for gene transfer which increases the efficiency of gene transfer into a host cell.

### BACKGROUND ART

Methods for introducing a gene of interest into a cultured cell to observe change of the cell are used, for example, when analyzing the function of a gene. Such gene transfer methods have become significantly popular because they can be more easily carried out and are more inexpensive compared to methods for preparing a transgenic animal.

Many methods for gene transfer into a cultured cell are known. Examples thereof include an electroporation method, a liposome method (lipoplex method), a polyplex method, a peptide method and a virus vector method. In particular, a liposome method, polyplex method, etc., in which a gene is introduced into a host cell using a reagent which has affinity to both the gene to be introduced and the host cell, are often used because of efficiency and easiness (for example, see: Gao X et al., Cationic liposome mediated gene transfer., Gene Ther., vol. 2, pp. 710-722, 1995; and J Rejman et al., Role of clathrin and caveolae mediated endocytosis in gene transfer mediated by lipo and polyplex., Molecular therapy, vol. 12, pp. 468-474, 2005).

However, when employing a conventional gene transfer method, the transfer efficiency often varies depending on the type of host cell. Further, some reagents have cytotoxicity. Therefore, when using such a reagent, it cannot be added in a sufficient amount, and as a result, it is difficult to efficiently perform gene transfer. For this reason, the progress of experiments and researches is often hindered.

### DISCLOSURE OF THE INVENTION

The problem to be solved by the present invention is to provide a means for introducing a gene of interest into a host cell with a higher degree of efficiency regardless of the gene transfer method employed and the type of the host cell, and to provide a gene transfer method using the means and a kit for gene transfer.

The present inventor diligently made researches in order to solve the above-described problem and found that the above-described problem can be solved by using a specific peptide as an auxiliary reagent when introducing a gene of interest into a host cell, and thus the present invention was achieved.

More specifically, the present invention is as follows:
(1) An auxiliary reagent for gene transfer comprising a peptide of (a) or (b) below, a derivative thereof, or a salt of the peptide or the derivative:
   (a) a peptide comprising an amino acid sequence represented by the following formula (I):

      C-X-[D/N]-X-X-X-X-[F/Y]-X-C-X-C (I)

      wherein X represents any amino acid residue, C represents cysteine, [D/N] represents aspartic acid or asparagine, and [F/Y] represents phenylalanine or tyrosine;
   (b) a peptide, which comprises the amino acid sequence represented by the formula (I) having deletion, substitution or addition of one or several amino acids, and which has activity to increase the efficiency of gene transfer into a host cell.
(2) A DNA encoding a peptide of (a) or (b) below:
   (a) a peptide comprising an amino acid sequence represented by the following formula (I):

      C-X-[D/N]-X-X-X-X-[F/Y]-X-C-X-C (I)

      wherein X represents any amino acid residue, C represents cysteine, [D/N] represents aspartic acid or asparagine, and [F/Y] represents phenylalanine or tyrosine;
   (b) a peptide, which comprises the amino acid sequence represented by the formula (I) having deletion, substitution or addition of one or several amino acids, and which has activity to increase the efficiency of gene transfer into a host cell.
(3) A recombinant vector comprising the DNA according to item (2).
(4) A transformant comprising the recombinant vector according to item (3).
(5) A gene transfer method, wherein a gene is introduced into a host cell in the presence of the reagent according to item (1).
(6) A kit for gene transfer, comprising the reagent according to item (1).

### BRIEF DESCRIPTION OF THE DRAWINGS

FIG. 1 is a graph showing comparison of the activity to increase gene transfer efficiency in transfer of gene into vascular endothelial cell (Pro5) using jet-PEI based on the presence/absence of Del-1 protein.
FIG. 2 is a graph showing comparison of the activity to increase gene transfer efficiency in transfer of gene into vascular endothelial cell (Pro5) using Lipofectamine2000 based on the presence/absence of Del-1 protein.
FIG. 3 is a graph showing comparison of the activity to increase gene transfer efficiency in transfer of gene into renal epithelium-derived cell (Cos1) based on the presence/absence of Del-1 protein.
FIG. 4 is a graph showing comparison of the activity to increase gene transfer efficiency among different proteins.
FIG. 5 is a graph showing alkaline phosphatase activity in serum of wild type mouse (WT) and transgenic mouse (TG).

### BEST MODE FOR CARRYING OUT THE INVENTION

Hereinafter, the present invention will be described in detail. The scope of the present invention is not limited to the description. In addition to the following examples, the present invention can be suitably changed and then practiced within a range in which the effects of the present invention are not reduced.

Note that the entire specification of Japanese Patent Application No. 2006-276322, to which priority is claimed by the present application, is incorporated herein. In addition, all the publications such as prior art documents, laid-open publications, patents and other patent documents cited herein are incorporated herein by reference.

### 1. Auxiliary reagent for gene transfer

As described above, the auxiliary reagent for gene transfer of the present invention (hereinafter referred to as the auxiliary reagent of the present invention) comprises a peptide of the following (a):
(a) a peptide comprising an amino acid sequence represented by the following formula (I):

   C-X-[D/N]-X-X-X-X-[F/Y]-X-C-X-C (SEQ ID NO: 1) (I)

In the above-described peptide in (a), the amino acid sequence of formula (I) is described using one-character codes. For example, C, D, N, F and Y mean cysteine (Cys), aspartic acid (Asp), asparagine (Asn), phenylalanine (Phe) and tyrosine (Tyr), respectively. [D/N] represents aspartic acid or asparagine, and [F/Y] represents phenylalanine or tyrosine. X represents any amino acid residue, which can be generally selected from 20 types of amino acid residues, i.e., A (Ala), R (Arg), D (Asp), . N (Asn), C (Cys), Q (Gln), E (Glu), G (Gly), H (His), I (Ile), L (Leu), K (Lys), M (Met), F (Phe), P (Pro), S (Ser), T (Thr), W (Trp), Y (Tyr) and V (Val). In the present specification, other amino acid sequences may also be described using one-character codes like the case of formula (I).

The aforementioned amino acid sequence of formula (I) includes the following amino acid sequences represented by SEQ ID NOS: 2 to 5. Among them, the amino acid sequence represented by SEQ ID NO: 2 is preferred.
C-X-D-X-X-X-X-Y-X-C-X-C (SEQ ID NO: 2)
C-X-D-X-X-X-X-F-X-C-X-C (SEQ ID NO: 3)
C-X-N-X-X-X-X-Y-X-C-X-C (SEQ ID NO: 4)
C-X-N-X-X-X-X-F-X-C-X-C (SEQ ID NO: 5)

Specifically, preferred examples of the amino acid sequence of formula (I) are the following amino acid sequences represented by SEQ ID NOS: 6 and 7, and the amino acid sequence represented by SEQ ID NO: 6 is more preferred.
C-T-[D/N]-L-V-A-N-[F/Y]-S-C-E-C (SEQ ID NO: 6)
C-K-[D/N]-D-I-S-S-[F/Y]-E-C-W-C (SEQ ID NO: 7)

As used herein, "peptide" refers to a structure in which at least 2 amino acids are bound together by peptide bond. Examples thereof include oligopeptide and polypeptide. A substance in which a given three-dimensional structure is formed by polypeptides is called "protein". In the present invention, protein of this kind is also included in the above-described "peptide". Therefore, the peptide included in the auxiliary reagent of the present invention may be any of oligopeptide, polypeptide and protein.

As described above, the auxiliary reagent of the present invention may comprise a peptide of the following (b):
(b) a peptide, which comprises the amino acid sequence represented by the formula (I) having deletion, substitution or addition of one or several amino acids, and which has activity to increase the efficiency of gene transfer into a host cell.

In this regard, examples of the above-described "amino acid sequence having deletion, substitution or addition of one or several amino acids" include, but are not limited to, amino acid sequences having deletion, substitution or addition of about 1 to 5 amino acids, and preferably about 1 to 2 amino acids. In the present invention, the amino acid sequence represented by the formula (I) without having deletion, substitution or addition of amino acid residues at positions 1, 3, 8, 10 and 12 (C, [D/N], [F/Y], C and C in this order), and/or the amino acid sequence represented by the formula (I) without having deletion or addition of amino acid residues at positions 2, 4-7, 9 and 11 (all of them are X) are preferred. The introduction of mutation such as deletion, substitution and addition can be carried out using a kit for introducing mutation utilizing site-directed mutagenesis such as GeneTailor^{™} Site-Directed Mutagenesis System (manufactured by Invitrogen Corporation) and TaKaRa Site-Directed Mutagenesis System (Mutan-K, Mutan-Super Express K, etc.: manufactured by Takara Bio Inc.). Further, whether or not a peptide has the above-described introduced mutation (deletion, substitution or addition) can be confirmed using various amino acid sequence determination methods, structural analysis methods using X-ray, NMR and the like, etc.

In the present invention, activity to increase the efficiency of gene transfer means enhancement of endocytosis and phagocytosis of the host cell. The activity can be measured, for example, by means of reporter assay using a reporter gene such as β-galactosidase, luciferase gene, chloramphenicol acetyltransferase (CAT) gene and GFP or mutant thereof (EGFP, YFP, BFP, etc.) as a gene to be introduced.

As long as the aforementioned peptide of (a) or (b) included in the auxiliary reagent of the present invention is a peptide comprising the amino acid sequence represented by the formula (I) or a peptide comprising the amino acid sequence having the aforementioned deletion, substitution or addition, the number of residues of constituent amino acids of the peptide is not limited. 12 or more residues are preferred. For example, the peptide may have 12 to 100 residues, 12 to 50 residues, or 12 to 30 residues.

The aforementioned peptide of (a) or (b) may be a peptide from a natural product or a peptide obtained by chemical synthesis. There is no limitation regarding this, but peptides from natural products are preferred since many of such peptides do not exert adverse effects such as cytotoxicity.

Examples of peptides from natural products include naturally-occurring oligopeptides, polypeptides and proteins, and fragments thereof. The peptide from a natural product can be directly obtained from the natural product using a publicly-known collection method and purification method. Alternatively, a gene encoding the peptide is incorporated into any of various expression vectors or the like and introduced into a cell by means of a publicly-known genetic engineering technique, and after expressed, the peptide may be obtained by means of a publicly-known collection method and purification method. Alternatively, the peptide is produced by the cell-free protein synthesizing system using a commercially-available kit such as reagent kits PROTEIOS^{™} (Toyobo Co., Ltd.) and TNT^{™} System (Promega), a synthesis device PG-Mate^{™} (Toyobo Co., Ltd.), RTS (Roche Diagnostics K.K.), etc., and then the peptide may be obtained by means of a publicly-known collection method and purification method. There is no limitation regarding this.

The chemically-synthesized peptide can be obtained using a publicly-known peptide synthesis method. Examples of the synthesis methods include azide method, acid chloride method, acid anhydride method, mixed acid anhydride method, DCC method, active ester method, carboimidazole method and oxidation-reduction method. To such synthesis, solid-phase synthesis or liquid-phase synthesis may be applied. A commercially-available peptide synthesis apparatus may be used. After synthetic reaction, the peptide can be purified using a combination of publicly-known purification methods such as chromatography.

Hereinafter, as examples of peptides, which are the aforementioned peptide of (a) or (b), and which correspond to a peptide from a natural product, (i) developmentally regulated endothelial cell locus-1 (Del-1: developmentally endothelial locus-1) protein and a partial fragment thereof and (ii) blood coagulation factor IX will be described.

Del-1 protein is an extracellular matrix protein having epidermal growth factor (EGF)-like domains and discoidin I-like domains, which consists of the amino acid sequence represented by SEQ ID NO: 10. It is known that EGF1, EGF2 and EGF3 exist as EGF-like domains of Del-1 protein, and that Discoidin1 and Discoidin2 exist as discoidin I-like domains. Among the above-described various domains, the present inventor focused their attention on EGF3, and further focused their attention on a region consisting of specific 12 amino acid residues in the amino acid sequence constituting EGF3 (SEQ ID NO: 12) (C-T-D-L-V-A-N-Y-S-C-E-C (SEQ ID NO: 8)). Further, the present inventor found that peptides including the specific region have activity to increase the efficiency of gene transfer. That is, examples of peptides, which are the aforementioned peptide of (a) or (b), and which correspond to a peptide from a natural product, include full-length Del-1 protein, partial fragment of Del-1 protein consisting of EGF3, and various partial fragments of Del-1 protein including EGF3 (see Table 1). Regarding the details of Del-1 protein and partial fragments thereof, the entire description in International Publication WO2005/0001093 pamphlet can be referred to. Partial fragments other than those having any of the amino acid sequences shown in Table 1 can be included in the present invention. For example, a partial fragment having an amino acid sequence in which a signal peptide portion is deleted from "full-length Del-1" in Table 1 can also be included in the present invention. The above-described "amino acid sequence in which a signal peptide portion is deleted from full-length Del-1" is an amino acid sequence encoded by a nucleotide sequence consisting of bases at positions 694-2061 in the nucleotide sequence represented by SEQ ID NO: 9, i.e., an amino acid sequence consisting of amino acid residues at positions 26-480 in the amino acid sequence represented by SEQ ID NO: 10.

**Table 1**

| Name of fragment or the like | Region * | Type | SEQ ID NO: |
|---|---|---|---|
| Full-length Del-1 | 619-2061 | DNA | 9 |
| | 1-480 | Protein | 10 |
| EGF3 | 985-1089 | DNA | 11 |
| | 123-157 | Protein | 12 |
| 4-1 | 619-1662 | DNA | 13 |
| | 1-348 | Protein | 14 |
| 4-15 | 619-1713 | DNA | 15 |
| | 1-365 | Protein | 16 |
| 4-14 | 619-1722 | DNA | 17 |
| | 1-368 | Protein | 18 |
| 4-13 | 619-1773 | DNA | 19 |
| | 1-385 | Protein | 20 |
| XY | 985-1662 | DNA | 21 |
| | 123-348 | Protein | 22 |
| XC | 985-1269 | DNA | 23 |
| | 123-217 | Protein | 24 |
| human XY | - | DNA | 25 |
| | - | Protein | 26 |

| | | | |
|---|---|---|---|
| * Regions in DNAs are indicated by base numbers, and regions in proteins are indicated by amino acid numbers. Note that base numbers and amino acid numbers used herein are corresponding numbers on the full-length Del-1 sequence. That is, in the case of base numbers, they are base numbers on the nucleotide sequence represented by SEQ ID NO: 9, and in the case of amino acid sequences, numbers of amino acid residues on the amino acid sequence represented by SEQ ID NO: 10 are used. | | | |

Blood coagulation factor IX is a protein consisting of the amino acid sequence represented by SEQ ID NO: 28, and it is a single-stranded glycoprotein having Gla domain, EGF domain and protease domain in this order from the N-terminus. In this regard, the EGF domain of the factor IX is constituted by a sequence (SEQ ID NO: 30) consisting of amino acids at positions 90-123 in the amino acid sequence represented by SEQ ID NO: 28. The present inventor focused their attention on the fact that a region consisting of specific 12 amino acid residues (C-K-D-D-1-S-S-Y-E-C-W-C (SEQ ID NO: 31)) is included in the amino acid sequence constituting the EGF domain of the factor IX as in the case of the aforementioned EGF3 of Del-1 protein (EGF-like domain), and found that peptides including this region can increase the efficiency of gene transfer. That is, examples of peptides, which are the aforementioned peptide of (a) or (b), and which correspond to a peptide from a natural product, include full-length factor IX, peptide fragment consisting of the amino acid sequence represented by SEQ ID NO: 31, various partial fragments including the fragment, partial fragment consisting of the EGF domain, and various partial fragments including the EGF domain. The nucleotide sequence of DNA encoding the full-length factor IX is represented by SEQ ID NO: 27, and the nucleotide sequence of DNA encoding the EGF domain of the factor IX is represented by SEQ ID NO: 29.

The auxiliary reagent of the present invention may include a derivative of the aforementioned peptide of (a) or (b) together with or instead of the peptide. The "derivative" means that all derivatives which can be prepared from the aforementioned peptide of (a) or (b) are included. Examples thereof include a derivative in which a part of constituent amino acids of the peptide are replaced by nonnatural amino acids and a derivative in which a part of constituent amino acids of the peptide (mainly side chain thereof) are chemically modified.

The auxiliary reagent of the present invention may include a salt of the aforementioned peptide of (a) or (b) and/or a salt of a derivative of the peptide together with or instead of the peptide and/or the derivative. These salts are preferably physiologically-acceptable acid addition salts or basic salts. Examples of acid addition salts include salts using inorganic acids such as hydrochloric acid, phosphoric acid, hydrobromic acid and sulfuric acid, and salts using organic acids such as acetic acid, formic acid, propionic acid, fumaric acid, maleic acid, succinic acid, tartaric acid, citric acid, malic acid, oxalic acid, benzoic acid, methanesulfonic acid and benzenesulfonic acid. Examples of basic salts include salts using inorganic bases such as sodium hydroxide, potassium hydroxide, ammonium hydroxide and magnesium hydroxide, and salts using organic bases such as caffeine, piperidine, trimethylamine and pyridine.

Salts can be prepared using a suitable acid such as hydrochloric acid or a suitable base such as sodium hydroxide. For example, salts can be prepared by treatment in water or a solution containing an inactive water-miscible organic solvent such as methanol, ethanol and dioxane using a standard protocol.

The auxiliary reagent of the present invention may consist of the above-described peptide of (a) or (b), a derivative thereof, or a salt of the peptide or the derivative, or may include: the peptide, a derivative thereof, or a salt of the peptide or the derivative; and other components. There is no limitation regarding this. Examples of other components include buffer solutions such as PBS and Tris-HCl, and additives such as sodium azide and glycerol. When the auxiliary reagent of the present invention includes other components, each of the contents thereof can be suitably determined within a range in which the activity to increase the efficiency of gene transfer provided by the above-described peptide, a derivative thereof, or a salt of the peptide or the derivative is not significantly blocked.

### 2. DNA

The DNA of the present invention includes DNAs consisting of a nucleotide sequence encoding an amino acid sequence constituting the aforementioned peptide of (a) or (b). Therefore, the DNA of the present invention may consist of only a DNA consisting of a nucleotide sequence encoding the aforementioned peptide of (a) or (b), or may include the DNA and another publicly-known nucleotide sequence required for gene expression (transcription promoter, SD sequence, Kozak sequence, terminator, etc.). There is no limitation regarding this. In the nucleotide sequence encoding the aforementioned peptide of (a) or (b), the type of codon is not limited. For example, after transcription, a codon which is generally used in mammals such as human may be used, or a codon which is generally used in microorganisms such as E. coli and yeast, plants or the like may be used. This can be suitably selected or designed.

In addition, in the present invention, a DNA, which can hybridize to a DNA consisting of a nucleotide sequence complementary to a DNA including a nucleotide sequence encoding the aforementioned peptide of (a) or (b) under stringent conditions, and which encodes a protein having activity to increase the efficiency of gene transfer, can also be included in the DNA of the present invention. In this regard, the term "stringent conditions" refers to, for example, conditions in which the concentration of salt (sodium) is 150 to 900 mM and the temperature is 55 to 75°C, and preferably conditions in which the concentration of salt (sodium) is 150 to 200 mM and the temperature is 60 to 70°C.

### 3. Recombinant vector and Transformant

### (1) Recombinant vector including DNA

The recombinant vector of the present invention can be obtained by linking (inserting) the DNA of the present invention to (into) a suitable vector. A vector into which the DNA of the present invention is to be inserted is not particularly limited as long as it can be replicated in a host. Examples thereof include plasmid DNA, phage DNA, virus, etc.

Examples of plasmid DNAs include E. coli-derived plasmid, Bacillus subtilis-derived plasmid and yeast-derived plasmid. Examples of phage DNAs include λ phage. Examples of viruses include adenovirus and retrovirus.

Other than promoters and the DNA of the present invention, if desired, cis elements such as enhancers, splicing signals, poly(A) addition signals, selection markers, ribosome binding sequences (SD sequences) or the like may be linked to the vector of the present invention. Examples of selection markers include dihydrofolate reductase gene, ampicillin resistance gene and neomycin resistance gene.

### (2) Transformant

The transformant of the present invention can be obtained by introducing the recombinant vector of the present invention into a host so that a gene of interest can be expressed. The host is not limited as long as it can express the DNA of the present invention. Examples of hosts which may be used in the invention include bacteria, yeasts and animal cells well-known in the art. Alternatively, experimental animals such as mouse may be used.

When a bacterium is used as a host, the recombinant vector of the invention is capable of autonomous replication in the bacterium, and at the same time, may also comprise a promoter, a ribosome binding sequence, the DNA of the invention and a transcription termination sequence. Specific examples of bacteria which may be used in the invention include E. coli (*Escherichia coli*). Examples of promoters which may be used in the invention include lac promoter. Examples of methods for introducing a vector into a bacterium include calcium ion method.

When an yeast is used as a host, *Saccharomyces cerevisiae* or the like may be used. A promoter which may be used in this case is not particularly limited as long as it allows expression in yeast. Examples thereof include gall promoter. Examples of methods for introducing a vector into an yeast include an electroporation method and spheroplast method.

When an animal cell is used as a host, CHO cells, rat GH3 cells, human HEK293 cells or the like may be used. Examples of promoters which may be used in the invention include SV40 promoter. Examples of methods for introducing a vector into an animal cell include an electroporation method and calcium phosphate method.

Further, for gene transfer into an animal or plant, a virus vector method, lipofection method or the like may be used. It is also possible to introduce a gene into a germ cell or ES cell to create a genetically modified animal.

### 4. Gene transfer method

As described above, the gene transfer method of the present invention is **characterized in that** a gene is introduced into a host cell in the presence of the auxiliary reagent of the present invention.

Specifically, the method of the present invention is **characterized in that**, in a reaction of gene transfer into a host cell using any of various publicly-known gene transfer methods such as a liposome method (lipoplex method), a polyplex method, a peptide method, an electroporation method and a virus vector method, the auxiliary reagent of the present invention is caused to exist in the same reaction system at least at the time of gene transfer. Therefore, as long as the auxiliary reagent of the present invention is caused to exist as described above, it may be added to the reaction system together with various gene transfer reagents and/or a gene of interest to be introduced, or these substances may be added separately. There is no limitation regarding this.

As long as the auxiliary reagent of the present invention is used in a manner as described above, the method of the present invention include any step. There is no limitation regarding this, but in general, steps according to protocols of various publicly-known gene introduction methods are preferably included.

The method of the present invention using the auxiliary reagent of the present invention can increase the efficiency of gene transfer into a host cell more effectively compared to the cases where various gene transfer methods are carried out according to general protocols. In addition, the method of the present invention can increase the efficiency of gene transfer regardless of the type of host cell to be used.

### 5. Kit for gene transfer

As described above, the kit for gene transfer of the present invention is **characterized in that** it includes the auxiliary reagent of the present invention as a constituent.

When a gene is introduced into various host cells using various publicly-known gene transfer methods, the kit of the present invention can further increase the transfer efficiency. Therefore, the kit of the present invention is significantly useful in the fields of experiments, researches, etc.

The kit of the present invention may include constituents other than the auxiliary reagent of the present invention. Examples of other constituents include, but are not limited to, buffer solutions such as PBS and Tris-HCl, additives such as sodium azide and glycerol, an experiment manual, a gene transfer reagent, etc.

### EXAMPLES

Hereinafter, the present invention will be specifically described by way of illustrative examples, but the present invention is not limited thereto.

### [Example 1]

### <Increase of transfer efficiency in gene transfer method using polyplex method>

Experiment of transfer of lacZ gene into a Pro5 cell (vascular endothelial cell) was carried out using a gene transfer reagent jet-PEI (Polyplus Transfection) in which linear polyethyleneimine (PEI), which is a cationic water-soluble polymer, is based.

Firstly, using a pAPtag-4 vector (GenHunter), an expression vector, which includes a DNA encoding a fusion protein of alkaline phosphatase and Del-1 (excluding the signal peptide portion), was constructed as follows. The pAPtag-4 vector as used herein can transiently express a protein in which a signal peptide and alkaline phosphatase are fused to the N-terminus of a protein of interest by inserting a DNA encoding the protein of interest into a predetermined position.

An RNA was extracted from a mouse embryo on day 9 to day 12 after fertilization using TRIzol (Invitrogen Corporation). Using this RNA as a template, a reverse transcription reaction was performed according to the ordinary method utilizing Superscrpt II (Invitrogen Corporation) to prepare a cDNA.

Using this cDNA as a template, a nucleotide sequence encoding an amino acid sequence in which the signal peptide portion is deleted from the amino acid sequence of the full-length Del-1 represented by SEQ ID NO: 10, i.e., a nucleotide sequence consisting of bases at positions 694 to 2061 in the nucleotide sequence represented by SEQ ID NO: 9, was amplified by means of PCR. In order to enable insertion of the amplified product into the pAPtag-4 vector, F and R primers, in which a restriction enzyme recognition sequence was introduced to the 5' end, were synthesized.

F primer:
5'- tgcaacccgaacccctgtgaaaatggt -3' (SEQ ID NO: 32)

R primer:
5'- ttcctcctctgcgcagcccagcagc -3' (SEQ ID NO: 33)

The above-described PCR was performed using a reaction solution with the following composition under the following reaction conditions.

| <Composition of reaction solution> | |
|---|---|
| Template DNA (50 ng/µL) | |
| (cDNA produced by a reverse transcriptase): | 5 µL |
| F primer (50 pM): | 1 µL |
| R primer (50 pM): | 1 µL |
| dNTP (2.5 mM for each): | 4 µL |
| Taq polymerase (5 unit/µL): | 1 µL |
| Buffer: | 5 µL |
| (20mM Tris-HCl (pH8.3), 100mM KCI, 20mM MgCl₂) | |
| Sterile water: | Appropriate amount (about 33 µL) |
| Total: | 50 µL |

### <Reaction conditions>

In one cycle, reaction conditions are as follows: "at 94°C for 30 seconds (heat denaturation/disassociation) → at 62°C for 30 seconds (annealing) → at 72°C for 90 seconds (synthesis/extension)". In total, 35 cycles were carried out.

After performing PCR, both the amplified fragment and the pAPtag-4 vector were treated with Bgl II and Xho I. After that, the amplified fragment was inserted into the pAPtag-4 vector using the publicly-known genetic engineering technique to construct an expression vector which can express a fusion protein of interest.

Next, the expression vector was introduced into a Cos1 cell using the ordinary method to prepare a conditioned medium. As a negative control (Dell(-)), only the pAPtag-4 vector was used. Next, according to the instructions attached to jet-PEI, a Pro5 cell and cDNA of a lacZ gene were prepared. To a culture solution at the time of introduction of lacZ gene, a fusion protein of alkaline phosphatase and Del-1 was added (Del-1 (+)), and to the negative control, alkaline phosphatase in an amount to have alkaline phosphatase activity equivalent to that of the above-described added fusion protein was added. 72 hours after the introduction of lacZ gene, the Pro5 cell was collected to measure β-galactosidase activity.

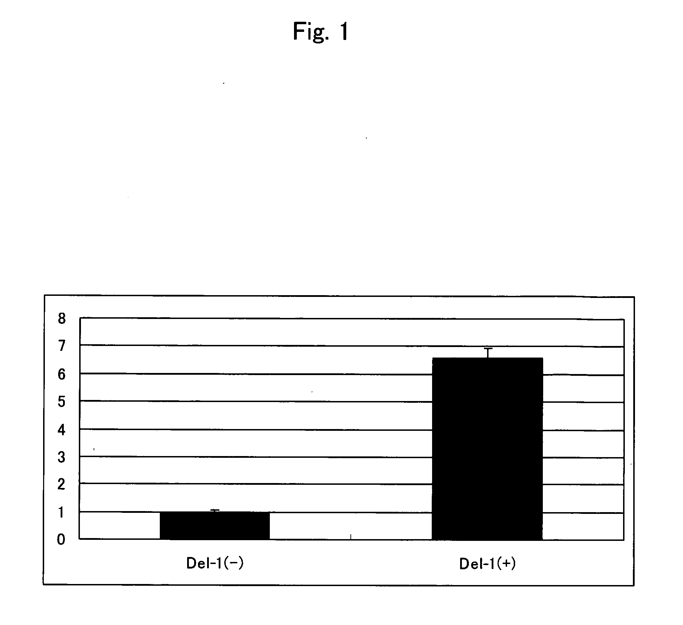

Results of the experiment are shown in Figure 1.

Because of the addition of the fusion protein of Del-1, the efficiency of gene transfer was successfully increased by about 6.5 times.

### [Example 2]

### <Increase of transfer efficiency in gene transfer method using liposome method>

Experiment of transfer of lacZ gene into a Pro5 cell (vascular endothelial cell) was carried out using a gene transfer reagent Lipofectamine 2000 (Invitrogen Corporation) in which cationic lipid is based.

Firstly, in a manner similar to that in Example 1, using a pAPtag-4 vector (GenHunter), an expression vector, which includes a DNA encoding a fusion protein of alkaline phosphatase and Del-1 (excluding the signal peptide portion), was constructed, and the expression vector was introduced into a Cos1 cell to prepare a conditioned medium. As a negative control, only the pAPtag-4 vector was used. Next, according to the instructions attached to Lipofectamine 2000, a Pro5 cell and cDNA of a lacZ gene were prepared. To a culture solution at the time of introduction of lacZ gene, a fusion protein of alkaline phosphatase and Del-1 was added (Del-1 (+)), and to the negative control (Del-1(-)), alkaline phosphatase in an amount to have alkaline phosphatase activity equivalent to that of the above-described added fusion protein was added. 72 hours after the introduction of lacZ gene, the Pro5 cell was collected to measure β-galactosidase activity.

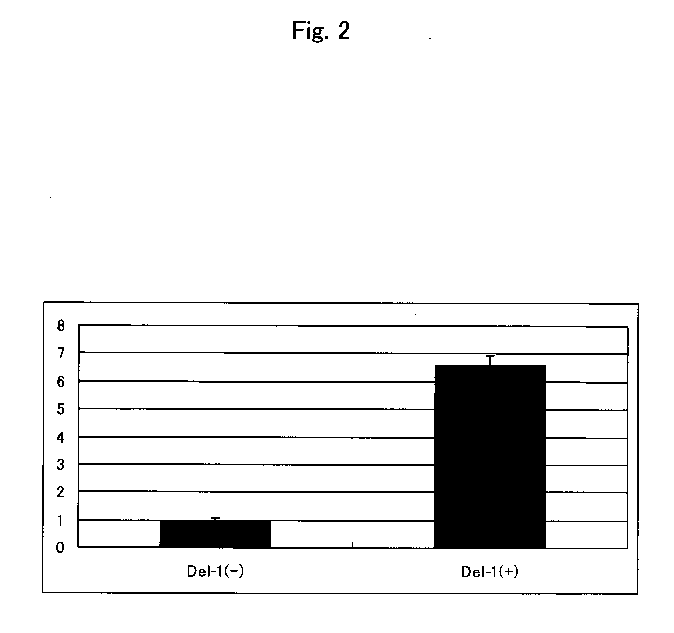

Results of the experiment are shown in Figure 2.

Because of the addition of the fusion protein of Del-1, the efficiency of gene transfer was successfully increased by about 15 times.

### [Example 3]

### <Increase of gene transfer efficiency with respect to renal epithelium-derived cell (Cos1)>

### Experiment of transfer of lacZ gene into a Cos1 cell was carried out using jet-PEI (Polyplus Transfection).

Firstly, in a manner similar to that in Example 1, using a pAPtag-4 vector (GenHunter), an expression vector, which includes a DNA encoding a fusion protein of alkaline phosphatase and Del-1 (excluding the signal peptide portion), was constructed, and the expression vector was introduced into a Cos1 cell to prepare a conditioned medium. As a negative control (Del-1 (-)), only the pAPtag-4 vector was used. Next, according to the instructions attached to jet-PEI, a Cos1 cell and cDNA of a lacZ gene were prepared. To a culture solution at the time of introduction of lacZ gene, a fusion protein of alkaline phosphatase and Del-1 was added (Del-1 (+)), and to the negative control, alkaline phosphatase in an amount to have alkaline phosphatase activity equivalent to that of the above-described added fusion protein was added. 72 hours after the introduction of lacZ gene, the Pro5 cell was collected to measure β-galactosidase activity.

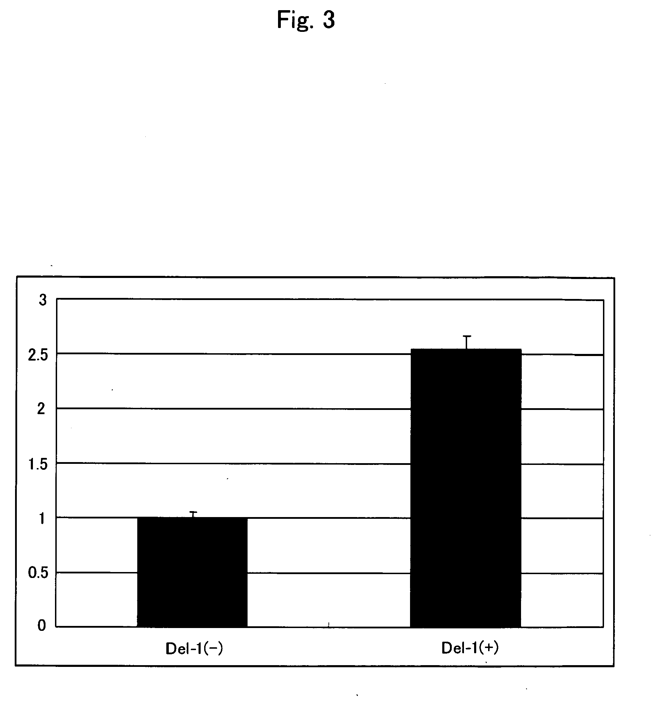

Results of the experiment are shown in Figure 3.

With respect to the effect of increasing the gene transfer efficiency by addition of the fusion protein of Del-1, when using the Pro5 cell as a host cell (Example 1), the efficiency was increased by about 6.5 times, and when using the Cos1 cell in this example, the efficiency was increased by about 2.5 times. The apparent effect was small, but a system in which Cos1 cell is used as a host cell is a system by which high-efficiency gene transfer can be realized from the start. Therefore, since the high efficiency of introduction in a system using Cos1 cell was successfully increased further by about 2.5 times, it can be said that a significantly beneficial effect is exerted.

### [Example 4]

### <Examination regarding protein having activity to increase the efficiency of gene transfer>

Examination was made as to what kind of amino acid sequence in Del-1 has activity to increase the efficiency of gene transfer and whether or not there is an amino acid sequence of a protein other than Del-1 having the activity.

Specifically, each of the following 4 types of proteins (i) to (iv) was subjected to examination for comparison of activity to increase the gene transfer efficiency.
(i) Del-1 (excluding the signal peptide portion)
(ii) EGF3 domain of Del-1 (EGF 3rd of Del-1; SEQ ID NO: 11)
(iii) protein in which the amino acid at position 136 of EGF3 domain of Del-1, aspartic acid is replaced by glutamic acid (EGF 3rd of Del-1(D136E))
(iv) EGF domain of blood coagulation factor IX (F-IX)

Firstly, in a manner similar to that in Example 1, using a pAPtag-4 vector (GenHunter), expression vectors, each of which includes a DNA encoding a fusion protein of alkaline phosphatase and each of the above-described proteins, were constructed.

As DNAs encoding (ii) EGF 3rd of Del-1 or (iv) F-IX, products obtained by performing PCR similar to that in Example 1 were used except that the following primers were used, respectively.

Primers for obtaining DNA encoding (ii) EGF 3rd of Del-1

F primer:
5'- tgtgaagctgagccttgcagaaatgg -3' (SEQ ID NO: 34)

R primer:
5'- atattgacaatttcgtcccataaatt -3' (SEQ ID NO: 35)

Primers for obtaining DNA encoding (iv) F-IX

F primer:
5'- aaagatcttgtgaatcaaatccttgtttaaatgg -3' (SEQ ID NO: 36)

R primer:
5'- ttctcgagttaattcacagttccttccttcaaatc -3' (SEQ ID NO: 37)

As a DNA encoding (iii) EGF 3rd of Del-1(D136E), a product obtained by introducing mutation into the above-described DNA encoding (ii) EGF 3rd of Del-1 obtained by means of PCR using QuikChange kit (Stratagene) was used.

Next, each of the expression vectors constructed as described above was introduced into a Cos1 cell to prepare a conditioned medium. As a negative control, only the pAPtag-4 vector was used. To a culture solution at the time of introduction of lacZ gene, a fusion protein of alkaline phosphatase and each of the above-described proteins was added, and to the negative control, alkaline phosphatase in an amount to have alkaline phosphatase activity equivalent to that of the above-described added fusion protein was added. 72 hours after the introduction of lacZ gene, the Pro5 cell was collected to measure β-galactosidase activity.

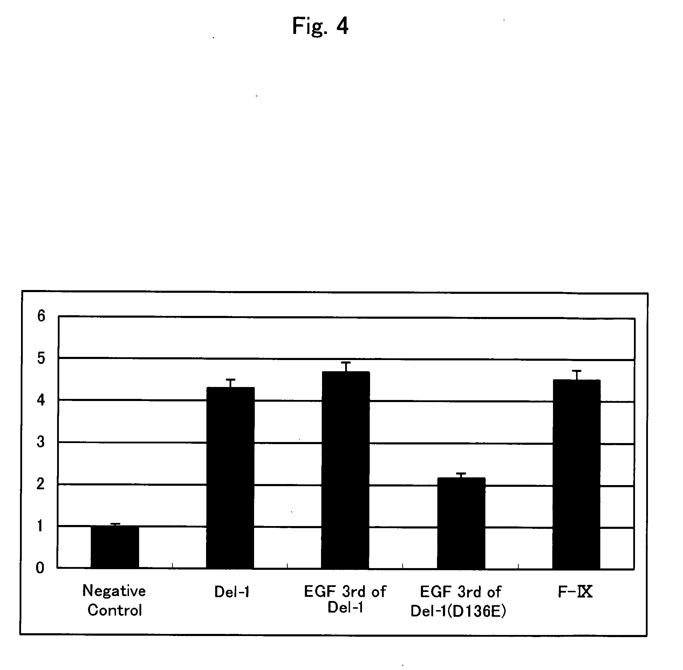

Results of the experiment are shown in Figure 4.

Since EGF3 of Del-1 solely exerted the same effect as that of Del-1, it was thought that the active center exists in EGF3. Based on already-published reports regarding other types of proteins, it was predicted that aspartic acid at position 136 of the amino acid sequence of EGF3 would be affected by β-hydroxylation. When aspartic acid in EGF3 was replaced by glutamic acid, the activity to increase the gene transfer efficiency was significantly suppressed. Thus, it was found that aspartic acid in EGF3 is an important amino acid. Further, EGF domain of coagulation factor IX, which is a naturally-occurring protein having a structure similar to that of EGF3 of Del-1, exerted high activity to increase the gene transfer efficiency as in the case of EGF3 of Del-1. Therefore, it was indicated that not only EGF3 of Del-1, but also family proteins comprising an amino acid sequence similar thereto have activity to increase the gene transfer efficiency.

### [Example 5]

### <Effect of promoting gene transfer by Del-1 in vivo>

Confirmation was made as to whether or not the peptide of the present invention has the effect of promoting gene transfer in vivo.

### (1) Method

According to the ordinary method, transgenic mice in which cDNA of full-length Del-1 (i.e., DNA consisting of the nucleotide sequence represented by SEQ ID NO: 9) is overexpressed were prepared (see Hidai et al., Anatomical Record A, vol. 287A, pp. 1165-1175). Del-1 protein produced in cells of the transgenic mice is a protein having the amino acid sequence represented by SEQ ID NO: 10 (including the signal peptide). Del-1 protein secreted from the cells to the outside thereof is a protein having an amino acid sequence consisting of amino acid residues at positions 26 to 480 in the amino acid sequence represented by SEQ ID NO: 10 (excluding the signal peptide). Using TransIT In Vivo Gene Delivery System (Mirus), the above-described transgenic mice were subjected to tail vein injection of human thermostable alkaline phosphatase gene. 24 hours later, blood was collected from the mice and serum was separated. After separation, the collected serum was subjected to heat treatment at 65°C for 30 minutes to inactivate endogenous alkaline phosphatase. Using 20 µl of serum after the heat treatment, the activity of exogenous alkaline phosphatase (human thermostable alkaline phosphatase) was measured. In the measurement of the activity, 20 µl of serum was mixed with 200 µl of 1mg/ml p-nitrophenyl phosphate, and after causing reaction at room temperature for 30 minutes to 1 hour, the absorbance at 405 nm was measured using a micrometer (BioRad).

As a control group, wild-type mice born of the same mother as the transgenic mice were employed. The wild-type mice were also subjected to injection of human thermostable alkaline phosphatase gene in a manner similar to that in the case of the transgenic mice. After that, collecting blood and the like and heat treatment were carried out to measure exogenous alkaline phosphatase activity.

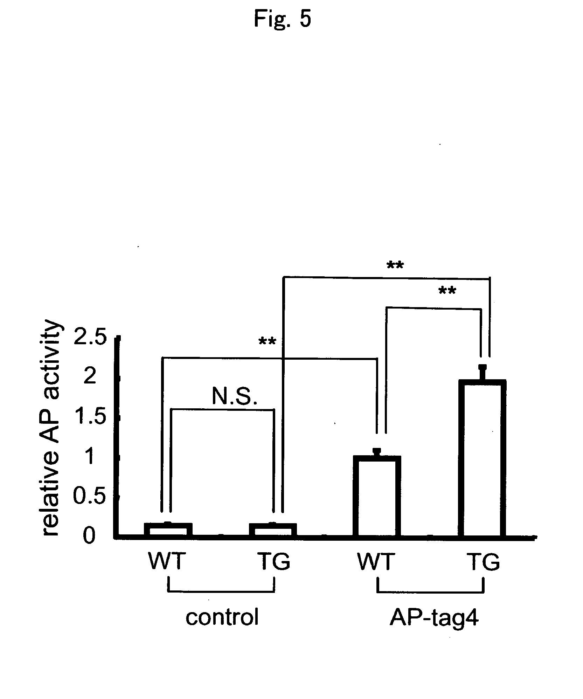

Measurement values of the alkaline phosphatase activity are expressed by average value ± SEM (standard error of the mean). The measurement values obtained from the control group into which alkaline phosphatase gene was not injected are regarded as 1, and the measurement values of the transgenic mice were calculated based thereon. Results are shown in Figure 5.

### (2) Results

Firstly, as shown by the left side (control) of the graph in Figure 5, in the case where the human thermostable alkaline phosphatase gene was not injected into the mice, it was confirmed that there is no difference between the endogenous alkaline phosphatase activity of the wild-type mouse (WT) and that of the transgenic mouse (TG).

Next, as shown by the right side (AP-tag4) of the graph in Figure 5, in the case where the human thermostable alkaline phosphatase gene was injected into the mice and collecting blood and the like and heat treatment were carried out as described above to measure exogenous alkaline phosphatase activity, the alkaline phosphatase activity of the transgenic mouse (TG) was about twice as high as that of the wild-type mouse (WT). Therefore, it was confirmed that the peptide of the present invention (full-length Del-1) increases the gene transfer efficiency of TransIT In Vivo Gene Delivery System.

### INDUSTRIAL APPLICABILITY

By utilizing the auxiliary reagent of the present invention, the gene transfer efficiency in various gene transfer methods can be significantly increased. The auxiliary reagent of the present invention can increase the gene transfer efficiency regardless of what kind of gene transfer method is used or what kind of host cell or transfer reagent is used, and therefore is excellent in terms of usefulness. Therefore, even in a cell in which only low transfer efficiency is realized by a commercially available gene transfer reagent, the transfer efficiency of a gene of interest can be dramatically increased, and accordingly, the efficiency of experiments, researches, etc. can be significantly improved.

### SEQUENCE LISTING FREE TEXT

SEQ ID NO: 1: Peptide
SEQ ID NO: 1: Xaa at Location: 3 represents aspartic acid (Asp) or asparagine (Asn), Xaa at Location: 8 represents phenylalanine (Phe) or tyrosine (Tyr), and Xaa at Locations: 2, 4, 5, 6, 7, 9 and 11 represents any amino acid residue.
SEQ ID NO: 2: Peptide
SEQ ID NO: 2: Xaa at Locations: 2, 4, 5, 6, 7, 9 and 11 represents any amino acid residue.
SEQ ID NO: 3: Peptide
SEQ ID NO: 3: Xaa at Locations: 2, 4, 5, 6, 7, 9 and 11 represents any amino acid residue.
SEQ ID NO: 4: Peptide
SEQ ID NO: 4: Xaa at Locations: 2, 4, 5, 6, 7, 9 and 11 represents any amino acid residue.
SEQ ID NO: 5: Peptide
SEQ ID NO: 5: Xaa at Locations: 2, 4, 5, 6, 7, 9 and 11 represents any amino acid residue.
SEQ ID NO: 6: Peptide
SEQ ID NO: 6: Xaa at Location: 3 represents aspartic acid (Asp) or asparagine (Asn), Xaa at Location: 8 represents phenylalanine (Phe) or tyrosine (Tyr), and Xaa at Locations: 2, 4, 5, 6, 7, 9 and 11 represents any amino acid residue.
SEQ ID NO: 7: Peptide
SEQ ID NO: 7: Xaa at Location: 3 represents aspartic acid (Asp) or asparagine (Asn), Xaa at Location: 8 represents phenylalanine (Phe) or tyrosine (Tyr), and Xaa at Locations: 2, 4, 5, 6, 7, 9 and 11 represents any amino acid residue.
SEQ ID NO: 32: Synthetic DNA
SEQ ID NO: 33: Synthetic DNA
SEQ ID NO: 34: Synthetic DNA
SEQ ID NO: 35: Synthetic DNA
SEQ ID NO: 36: Synthetic DNA
SEQ ID NO: 37: Synthetic DNA

## Claims

1. An auxiliary reagent for gene transfer comprising a peptide of (a) or (b) below, a derivative thereof, or a salt of the peptide or the derivative:
(a) a peptide comprising an amino acid sequence represented by the following formula (I):
C-X-[D/N]-X-X-X-X-[F/Y]-X-C-X-C (I)
wherein X represents any amino acid residue, C represents cysteine, [D/N] represents aspartic acid or asparagine, and [F/Y] represents phenylalanine or tyrosine;
(b) a peptide, which comprises the amino acid sequence represented by the formula (I) having deletion, substitution or addition of one or several amino acids, and which has activity to increase the efficiency of gene transfer into a host cell.

2. A DNA encoding a peptide of (a) or (b) below:
(a) a peptide comprising an amino acid sequence represented by the following formula (I):
C-X-[D/N]-X-X-X-X-[F/Y]-X-C-X-C (I)
wherein X represents any amino acid residue, C represents cysteine, [D/N] represents aspartic acid or asparagine, and [F/Y] represents phenylalanine or tyrosine;
(b) a peptide, which comprises the amino acid sequence represented by the formula (I) having deletion, substitution or addition of one or several amino acids, and which has activity to increase the efficiency of gene transfer into a host cell.

3. A recombinant vector comprising the DNA according to claim 2.

4. A transformant comprising the recombinant vector according to claim 3.

5. A gene transfer method, wherein a gene is introduced into a host cell in the presence of the reagent according to claim 1.

6. A kit for gene transfer, comprising the reagent according to claim 1.


SEQUENCE LISTING

<110> NIHON UNIVERSITY

<120> Adjunctive reagent for gene transfer

<130> G09-0017

<140> PCT/JP2007/070221
<141> 2007-10-10

<150> JP 2006-276322
<151> 2006-10-10

<160> 37

<170> PatentIn version 3.3

<210> 1
<211> 12
<212> PRT
<213> Artificial

<220>
<223> peptide


<220>
<221> MISC_FEATURE
<222> (2)..(2)
<223> Xaa can be any naturally occurring amino acid

<220>
<221> MISC_FEATURE
<222> (3)..(3)
<223> Xaa represents Asp or Asn

<220>
<221> MISC_FEATURE
<222> (4)..(7)
<223> Xaa can be any naturally occurring amino acid

<220>
<221> MISC_FEATURE

<222>  (8)..(8)
<223>  Xaa represents Phe or Tyr

<220>
<221>  MISC_FEATURE
<222>  (9)..(9)
<223>  Xaa can be any naturally occurring amino acid

<220>
<221>  MISC_FEATURE
<222>  (11)..(11)
<223>  Xaa can be any naturally occurring amino acid

<400>  1

Cys Xaa Xaa Xaa Xaa Xaa Xaa Xaa Xaa Cys Xaa Cys
1               5                   10


<210>  2
<211>  12
<212>  PRT
<213>  Artificial

<220>
<223>  peptide


<220>
<221>  MISC_FEATURE
<222>  (2)..(2)
<223>  Xaa can be any naturally occurring amino acid

<220>
<221>  MISC_FEATURE
<222>  (4)..(7)
<223>  Xaa can be any naturally occurring amino acid

<220>
<221>  MISC_FEATURE
<222>  (9)..(9)
<223>  Xaa can be any naturally occurring amino acid

<220>

<221>  MISC_FEATURE
<222>  (11)..(11)
<223>  Xaa can be any naturally occurring amino acid

<400>  2

Cys Xaa Asp Xaa Xaa Xaa Xaa Tyr Xaa Cys Xaa Cys
1               5                   10


<210>  3
<211>  12
<212>  PRT
<213>  Artificial

<220>
<223>  peptide


<220>
<221>  MISC_FEATURE
<222>  (2)..(2)
<223>  Xaa can be any naturally occurring amino acid

<220>
<221>  MISC_FEATURE
<222>  (4)..(7)
<223>  Xaa can be any naturally occurring amino acid

<220>
<221>  MISC_FEATURE
<222>  (9)..(9)
<223>  Xaa can be any naturally occurring amino acid

<220>
<221>  MISC_FEATURE
<222>  (11)..(11)
<223>  Xaa can be any naturally occurring amino acid

<400>  3

Cys Xaa Asp Xaa Xaa Xaa Xaa Phe Xaa Cys Xaa Cys
1               5                   10

<210> 4
<211> 12
<212> PRT
<213> Artificial

<220>
<223> peptide


<220>
<221> MISC_FEATURE
<222> (2)..(2)
<223> Xaa can be any naturally occurring amino acid

<220>
<221> MISC_FEATURE
<222> (4)..(7)
<223> Xaa can be any naturally occurring amino acid

<220>
<221> MISC_FEATURE
<222> (9)..(9)
<223> Xaa can be any naturally occurring amino acid

<220>
<221> MISC_FEATURE
<222> (11)..(11)
<223> Xaa can be any naturally occurring amino acid

<400> 4

Cys Xaa Asn Xaa Xaa Xaa Xaa Tyr Xaa Cys Xaa Cys
1               5                   10


<210> 5
<211> 12
<212> PRT
<213> Artificial

<220>
<223> peptide

<220>
<221> MISC_FEATURE
<222> (2)..(2)
<223> Xaa can be any naturally occurring amino acid

<220>
<221> MISC_FEATURE
<222> (4)..(7)
<223> Xaa can be any naturally occurring amino acid

<220>
<221> MISC_FEATURE
<222> (9)..(9)
<223> Xaa can be any naturally occurring amino acid

<220>
<221> MISC_FEATURE
<222> (11)..(11)
<223> Xaa can be any naturally occurring amino acid

<400> 5

Cys Xaa Asn Xaa Xaa Xaa Xaa Phe Xaa Cys Xaa Cys
1               5                   10

<210> 6
<211> 12
<212> PRT
<213> Artificial

<220>
<223> peptide

<220>
<221> MISC_FEATURE
<222> (3)..(3)
<223> Xaa represents Asp or Asn

<220>
<221> MISC_FEATURE
<222> (8)..(8)

<223>  Xaa represents Phe or Tyr

<400>  6

Cys Thr Xaa Leu Val Ala Asn Xaa Ser Cys Glu Cys
1               5                   10


<210>  7
<211>  12
<212>  PRT
<213>  Artificial

<220>
<223>  peptide


<220>
<221>  MISC_FEATURE
<222>  (3)..(3)
<223>  Xaa represents Asp or Asn

<220>
<221>  MISC_FEATURE
<222>  (8)..(8)
<223>  Xaa represents Phe or Tyr

<400>  7

Cys Lys Xaa Asp Ile Ser Ser Xaa Glu Cys Trp Cys
1               5                   10


<210>  8
<211>  12
<212>  PRT
<213>  Mus musculus

<400>  8

Cys Thr Asp Leu Val Ala Asn Tyr Ser Cys Glu Cys
1               5                   10

<210> 9
<211> 2303
<212> DNA
<213> Mus musculus

<220>
<221> CDS
<222> (619)..(2061)

<400> 9
gaattccggt taactgagga caaagggtaa tgcagaagtg atatttgatt tccattctca 60

ttcccagtgg ccttgatatt taaactgatt cctgccacca ggtccttggg ccaccctgtc 120

cctgcgtctc atatttctgc atgctgcttt gtttgtatat agtgcgctcc tggcctcagg 180

ctcgctcccc tccagctctc gcttcattgt tctccaagtc agaagccccc gcatccgccg 240

cgcagcagcg tgagccgtag tcactgctgg ccgcttcgcc tgcgtgcgcg cacggaaatc 300

ggggagccag gaacccaagg agccgccgtc cgcccgctgt gcctctgcta gaccactcgc 360

agccccagcc tctctcaagc gcacccacct ccgcgcaccc cagctcaggc gaagctggag 420

tgagggtgaa tcaccctttc tctagggcca ccactctttt atcgcccttc ccaagatttg 480

agaagcgctg cgggaggaaa gacgtcctct tgatctctga cagggcgggg tttactgctg 540

tcctgcaggc gcgcctcgcc tactgtgccc tccgctacga ccccggacca gcccaggtca 600

cgtccgtgag aagggatc atg aag cac ttg gta gca gcc tgg ctt ttg gtt 651
Met Lys His Leu Val Ala Ala Trp Leu Leu Val
1               5                   10

gga ctc agc ctc ggg gtg ccc cag ttc ggc aaa ggt gac att tgc aac 699
Gly Leu Ser Leu Gly Val Pro Gln Phe Gly Lys Gly Asp Ile Cys Asn
            15                  20                  25

ccg aac ccc tgt gaa aat ggt ggc atc tgt ctg tca gga ctg gct gat 747
Pro Asn Pro Cys Glu Asn Gly Gly Ile Cys Leu Ser Gly Leu Ala Asp
        30                  35                  40

gat tcc ttt tcc tgt gag tgt cca gaa ggc ttc gca ggt ccg aac tgc 795

Asp Ser Phe Ser Cys Glu Cys Pro Glu Gly Phe Ala Gly Pro Asn Cys
    45                  50                  55

tct agt gtt gtg gag gtt gca tca gat gaa gaa aag cct act tca gca     843
Ser Ser Val Val Glu Val Ala Ser Asp Glu Glu Lys Pro Thr Ser Ala
60                  65                  70                  75

ggt ccc tgc atc cct aac cca tgc cat aac gga gga acc tgt gag ata     891
Gly Pro Cys Ile Pro Asn Pro Cys His Asn Gly Gly Thr Cys Glu Ile
                80                  85                  90

agc gaa gcc tat cga gga gac aca ttc ata ggc tat gtt tgt aaa tgt     939
Ser Glu Ala Tyr Arg Gly Asp Thr Phe Ile Gly Tyr Val Cys Lys Cys
            95                  100                 105

cct cgg gga ttt aat ggg att cac tgt cag cac aat ata aat gaa tgt     987
Pro Arg Gly Phe Asn Gly Ile His Cys Gln His Asn Ile Asn Glu Cys
        110                 115                 120

gaa gct gag cct tgc aga aat ggc gga ata tgt acc gac ctt gtt gct     1035
Glu Ala Glu Pro Cys Arg Asn Gly Gly Ile Cys Thr Asp Leu Val Ala
    125                 130                 135

aac tac tct tgt gaa tgc cca gga gaa ttt atg gga cga aat tgt caa     1083
Asn Tyr Ser Cys Glu Cys Pro Gly Glu Phe Met Gly Arg Asn Cys Gln
140                 145                 150                 155

tat aaa tgc tct ggg cca ttg gga atc gaa ggt ggg atc ata tct aat     1131
Tyr Lys Cys Ser Gly Pro Leu Gly Ile Glu Gly Gly Ile Ile Ser Asn
                160                 165                 170

cag caa atc aca gct tca tct act cac cga gct ctt ttt gga ctc cgg     1179
Gln Gln Ile Thr Ala Ser Ser Thr His Arg Ala Leu Phe Gly Leu Arg
            175                 180                 185

aag tgg tat ccc tac tat gct cga ctt aat aag aag ggc ctt ata aat     1227
Lys Trp Tyr Pro Tyr Tyr Ala Arg Leu Asn Lys Lys Gly Leu Ile Asn
        190                 195                 200

gcc tgg aca gct gct gaa aat gac aga tgg cca tgg att cag ata aat     1275
Ala Trp Thr Ala Ala Glu Asn Asp Arg Trp Pro Trp Ile Gln Ile Asn
    205                 210                 215

ttg caa aga aaa atg aga gtc act ggt gtt att acc caa gga gca aaa     1323

Leu Gln Arg Lys Met Arg Val Thr Gly Val Ile Thr Gln Gly Ala Lys
220                 225                 230                 235

agg att gga agc cca gag tac ata aaa tcc tac aaa att gcc tac agc     1371
Arg Ile Gly Ser Pro Glu Tyr Ile Lys Ser Tyr Lys Ile Ala Tyr Ser
                240                 245                 250

aat gac ggg aag acc tgg gca atg tac aaa gta aaa ggc acc aat gaa     1419
Asn Asp Gly Lys Thr Trp Ala Met Tyr Lys Val Lys Gly Thr Asn Glu
            255                 260                 265

gag atg gtc ttt cgt gga aat gtt gat aac aac aca cca tat gct aat     1467
Glu Met Val Phe Arg Gly Asn Val Asp Asn Asn Thr Pro Tyr Ala Asn
        270                 275                 280

tct ttc aca ccc cca atc aaa gct cag tat gta aga ctc tac ccc caa     1515
Ser Phe Thr Pro Pro Ile Lys Ala Gln Tyr Val Arg Leu Tyr Pro Gln
    285                 290                 295

att tgt cga agg cat tgt act tta aga atg gaa ctt ctt ggc tgt gag     1563
Ile Cys Arg Arg His Cys Thr Leu Arg Met Glu Leu Leu Gly Cys Glu
300                 305                 310                 315

ctc tca ggc tgt tca gaa cct ttg ggg atg aaa tca ggg cat ata caa     1611
Leu Ser Gly Cys Ser Glu Pro Leu Gly Met Lys Ser Gly His Ile Gln
                320                 325                 330

gac tac cag atc act gcc tcc agc gtc ttc aga aca ctc aac atg gac     1659
Asp Tyr Gln Ile Thr Ala Ser Ser Val Phe Arg Thr Leu Asn Met Asp
            335                 340                 345

atg ttt act tgg gaa cca agg aaa gcc agg ctg gac aag caa ggc aaa     1707
Met Phe Thr Trp Glu Pro Arg Lys Ala Arg Leu Asp Lys Gln Gly Lys
        350                 355                 360

gta aat gcc tgg act tcc ggc cat aac gac cag tca caa tgg tta cag     1755
Val Asn Ala Trp Thr Ser Gly His Asn Asp Gln Ser Gln Trp Leu Gln
    365                 370                 375

gtt gat ctt ctt gtc cct act aag gtg aca ggc atc att aca caa gga     1803
Val Asp Leu Leu Val Pro Thr Lys Val Thr Gly Ile Ile Thr Gln Gly
380                 385                 390                 395

gct aaa gat ttt ggt cac gtg cag ttt gtt ggg tca tac aaa cta gct     1851

Ala Lys Asp Phe Gly His Val Gln Phe Val Gly Ser Tyr Lys Leu Ala
                400                 405                 410

tac agc aat gat gga gaa cac tgg atg gtg cac cag gat gaa aaa cag      1899
Tyr Ser Asn Asp Gly Glu His Trp Met Val His Gln Asp Glu Lys Gln
            415                 420                 425

agg aaa gac aag gtt ttt caa ggc aat ttt gac aat gac act cac agg      1947
Arg Lys Asp Lys Val Phe Gln Gly Asn Phe Asp Asn Asp Thr His Arg
        430                 435                 440

aaa aat gtc atc gac cct ccc atc tat gca cga ttc ata aga atc ctt      1995
Lys Asn Val Ile Asp Pro Pro Ile Tyr Ala Arg Phe Ile Arg Ile Leu
    445                 450                 455

cct tgg tcc tgg tat gga agg atc act ctg cgg tca gag ctg ctg ggc      2043
Pro Trp Ser Trp Tyr Gly Arg Ile Thr Leu Arg Ser Glu Leu Leu Gly
460                 465                 470                 475

tgc gca gag gag gaa tga agtgcggggc cgcacatccc acaatgcttt             2091
Cys Ala Glu Glu Glu
                480

tctttatttt cctataagta tctccacgaa atgaactgtg tgaagctgat ggaaactgca    2151

tttgtttttt tcaaagtgtt caaattatgg taggctactg actgtctttt taggagttct    2211

aagcttgcct ttttaataat ttaatttggt ttcctttgct caactctctt atgtaatatc    2271

acactgtctg tgagttactc ttcttgttct ct                                  2303

<210>  10
<211>  480
<212>  PRT
<213>  Mus musculus

<400>  10

Met Lys His Leu Val Ala Ala Trp Leu Leu Val Gly Leu Ser Leu Gly
1               5                   10                  15

Val Pro Gln Phe Gly Lys Gly Asp Ile Cys Asn Pro Asn Pro Cys Glu

            20                  25                  30

Asn Gly Gly Ile Cys Leu Ser Gly Leu Ala Asp Asp Ser Phe Ser Cys
        35                  40                  45

Glu Cys Pro Glu Gly Phe Ala Gly Pro Asn Cys Ser Ser Val Val Glu
    50                  55                  60

Val Ala Ser Asp Glu Glu Lys Pro Thr Ser Ala Gly Pro Cys Ile Pro
65                  70                  75                  80

Asn Pro Cys His Asn Gly Gly Thr Cys Glu Ile Ser Glu Ala Tyr Arg
                85                  90                  95

Gly Asp Thr Phe Ile Gly Tyr Val Cys Lys Cys Pro Arg Gly Phe Asn
            100                 105                 110

Gly Ile His Cys Gln His Asn Ile Asn Glu Cys Glu Ala Glu Pro Cys
        115                 120                 125

Arg Asn Gly Gly Ile Cys Thr Asp Leu Val Ala Asn Tyr Ser Cys Glu
    130                 135                 140

Cys Pro Gly Glu Phe Met Gly Arg Asn Cys Gln Tyr Lys Cys Ser Gly
145                 150                 155                 160

Pro Leu Gly Ile Glu Gly Gly Ile Ile Ser Asn Gln Gln Ile Thr Ala
                165                 170                 175

Ser Ser Thr His Arg Ala Leu Phe Gly Leu Arg Lys Trp Tyr Pro Tyr
            180                 185                 190

Tyr Ala Arg Leu Asn Lys Lys Gly Leu Ile Asn Ala Trp Thr Ala Ala

        195                 200                 205

Glu Asn Asp Arg Trp Pro Trp Ile Gln Ile Asn Leu Gln Arg Lys Met
    210                 215                 220

Arg Val Thr Gly Val Ile Thr Gln Gly Ala Lys Arg Ile Gly Ser Pro
225                 230                 235                 240

Glu Tyr Ile Lys Ser Tyr Lys Ile Ala Tyr Ser Asn Asp Gly Lys Thr
                245                 250                 255

Trp Ala Met Tyr Lys Val Lys Gly Thr Asn Glu Glu Met Val Phe Arg
            260                 265                 270

Gly Asn Val Asp Asn Asn Thr Pro Tyr Ala Asn Ser Phe Thr Pro Pro
        275                 280                 285

Ile Lys Ala Gln Tyr Val Arg Leu Tyr Pro Gln Ile Cys Arg Arg His
    290                 295                 300

Cys Thr Leu Arg Met Glu Leu Leu Gly Cys Glu Leu Ser Gly Cys Ser
305                 310                 315                 320

Glu Pro Leu Gly Met Lys Ser Gly His Ile Gln Asp Tyr Gln Ile Thr
                325                 330                 335

Ala Ser Ser Val Phe Arg Thr Leu Asn Met Asp Met Phe Thr Trp Glu
            340                 345                 350

Pro Arg Lys Ala Arg Leu Asp Lys Gln Gly Lys Val Asn Ala Trp Thr
        355                 360                 365

Ser Gly His Asn Asp Gln Ser Gln Trp Leu Gln Val Asp Leu Leu Val

370                 375                 380

Pro Thr Lys Val Thr Gly Ile Ile Thr Gln Gly Ala Lys Asp Phe Gly
385                 390                 395                 400

His Val Gln Phe Val Gly Ser Tyr Lys Leu Ala Tyr Ser Asn Asp Gly
                405                 410                 415

Glu His Trp Met Val His Gln Asp Glu Lys Gln Arg Lys Asp Lys Val
            420                 425                 430

Phe Gln Gly Asn Phe Asp Asn Asp Thr His Arg Lys Asn Val Ile Asp
        435                 440                 445

Pro Pro Ile Tyr Ala Arg Phe Ile Arg Ile Leu Pro Trp Ser Trp Tyr
    450                 455                 460

Gly Arg Ile Thr Leu Arg Ser Glu Leu Leu Gly Cys Ala Glu Glu Glu
465                 470                 475                 480

<210>  11
<211>  105
<212>  DNA
<213>  Mus musculus

<220>
<221>  CDS
<222>  (1)..(105)

<400>  11
tgt gaa gct gag cct tgc aga aat ggc gga ata tgt acc gac ctt gtt        48
Cys Glu Ala Glu Pro Cys Arg Asn Gly Gly Ile Cys Thr Asp Leu Val
1               5                   10                  15

gct aac tac tct tgt gaa tgc cca gga gaa ttt atg gga cga aat tgt        96
Ala Asn Tyr Ser Cys Glu Cys Pro Gly Glu Phe Met Gly Arg Asn Cys

            20                  25                  30

caa tat aaa 105
Gln Tyr Lys
        35

<210> 12
<211> 35
<212> PRT
<213> Mus musculus

<400> 12

Cys Glu Ala Glu Pro Cys Arg Asn Gly Gly Ile Cys Thr Asp Leu Val
1               5                   10                  15

Ala Asn Tyr Ser Cys Glu Cys Pro Gly Glu Phe Met Gly Arg Asn Cys
            20                  25                  30

Gln Tyr Lys
        35

<210> 13
<211> 1044
<212> DNA
<213> Mus musculus

<220>
<221> CDS
<222> (1)..(1044)

<400> 13
atg aag cac ttg gta gca gcc tgg ctt ttg gtt gga ctc agc ctc ggg 48
Met Lys His Leu Val Ala Ala Trp Leu Leu Val Gly Leu Ser Leu Gly
1               5                   10                  15

gtg ccc cag ttc ggc aaa ggt gac att tgc aac ccg aac ccc tgt gaa 96
Val Pro Gln Phe Gly Lys Gly Asp Ile Cys Asn Pro Asn Pro Cys Glu
            20                  25                  30

aat ggt ggc atc tgt ctg tca gga ctg gct gat gat tcc ttt tcc tgt 144
Asn Gly Gly Ile Cys Leu Ser Gly Leu Ala Asp Asp Ser Phe Ser Cys
        35                  40                  45

gag tgt cca gaa ggc ttc gca ggt ccg aac tgc tct agt gtt gtg gag 192
Glu Cys Pro Glu Gly Phe Ala Gly Pro Asn Cys Ser Ser Val Val Glu
    50                  55                  60

gtt gca tca gat gaa gaa aag cct act tca gca ggt ccc tgc atc cct 240
Val Ala Ser Asp Glu Glu Lys Pro Thr Ser Ala Gly Pro Cys Ile Pro
65                  70                  75                  80

aac cca tgc cat aac gga gga acc tgt gag ata agc gaa gcc tat cga 288
Asn Pro Cys His Asn Gly Gly Thr Cys Glu Ile Ser Glu Ala Tyr Arg
                85                  90                  95

gga gac aca ttc ata ggc tat gtt tgt aaa tgt cct cgg gga ttt aat 336
Gly Asp Thr Phe Ile Gly Tyr Val Cys Lys Cys Pro Arg Gly Phe Asn
            100                 105                 110

ggg att cac tgt cag cac aat ata aat gaa tgt gaa gct gag cct tgc 384
Gly Ile His Cys Gln His Asn Ile Asn Glu Cys Glu Ala Glu Pro Cys
        115                 120                 125

aga aat ggc gga ata tgt acc gac ctt gtt gct aac tac tct tgt gaa 432
Arg Asn Gly Gly Ile Cys Thr Asp Leu Val Ala Asn Tyr Ser Cys Glu
    130                 135                 140

tgc cca gga gaa ttt atg gga cga aat tgt caa tat aaa tgc tct ggg 480
Cys Pro Gly Glu Phe Met Gly Arg Asn Cys Gln Tyr Lys Cys Ser Gly
145                 150                 155                 160

cca ttg gga atc gaa ggt ggg atc ata tct aat cag caa atc aca gct 528
Pro Leu Gly Ile Glu Gly Gly Ile Ile Ser Asn Gln Gln Ile Thr Ala
                165                 170                 175

tca tct act cac cga gct ctt ttt gga ctc cgg aag tgg tat ccc tac 576
Ser Ser Thr His Arg Ala Leu Phe Gly Leu Arg Lys Trp Tyr Pro Tyr
            180                 185                 190

tat gct cga ctt aat aag aag ggc ctt ata aat gcc tgg aca gct gct 624
Tyr Ala Arg Leu Asn Lys Lys Gly Leu Ile Asn Ala Trp Thr Ala Ala
        195                 200                 205

gaa aat gac aga tgg cca tgg att cag ata aat ttg caa aga aaa atg     672
Glu Asn Asp Arg Trp Pro Trp Ile Gln Ile Asn Leu Gln Arg Lys Met
    210                 215                 220

aga gtc act ggt gtt att acc caa gga gca aaa agg att gga agc cca     720
Arg Val Thr Gly Val Ile Thr Gln Gly Ala Lys Arg Ile Gly Ser Pro
225                 230                 235                 240

gag tac ata aaa tcc tac aaa att gcc tac agc aat gac ggg aag acc     768
Glu Tyr Ile Lys Ser Tyr Lys Ile Ala Tyr Ser Asn Asp Gly Lys Thr
                245                 250                 255

tgg gca atg tac aaa gta aaa ggc acc aat gaa gag atg gtc ttt cgt     816
Trp Ala Met Tyr Lys Val Lys Gly Thr Asn Glu Glu Met Val Phe Arg
            260                 265                 270

gga aat gtt gat aac aac aca cca tat gct aat tct ttc aca ccc cca     864
Gly Asn Val Asp Asn Asn Thr Pro Tyr Ala Asn Ser Phe Thr Pro Pro
        275                 280                 285

atc aaa gct cag tat gta aga ctc tac ccc caa att tgt cga agg cat     912
Ile Lys Ala Gln Tyr Val Arg Leu Tyr Pro Gln Ile Cys Arg Arg His
    290                 295                 300

tgt act tta aga atg gaa ctt ctt ggc tgt gag ctc tca ggc tgt tca     960
Cys Thr Leu Arg Met Glu Leu Leu Gly Cys Glu Leu Ser Gly Cys Ser
305                 310                 315                 320

gaa cct ttg ggg atg aaa tca ggg cat ata caa gac tac cag atc act     1008
Glu Pro Leu Gly Met Lys Ser Gly His Ile Gln Asp Tyr Gln Ile Thr
                325                 330                 335

gcc tcc agc gtc ttc aga aca ctc aac atg gac atg                     1044
Ala Ser Ser Val Phe Arg Thr Leu Asn Met Asp Met
            340                 345

<210>  14
<211>  348
<212>  PRT
<213>  Mus musculus

<400>  14

Met Lys His Leu Val Ala Ala Trp Leu Leu Val Gly Leu Ser Leu Gly
1               5                   10                  15

Val Pro Gln Phe Gly Lys Gly Asp Ile Cys Asn Pro Asn Pro Cys Glu
            20                  25                  30

Asn Gly Gly Ile Cys Leu Ser Gly Leu Ala Asp Asp Ser Phe Ser Cys
        35                  40                  45

Glu Cys Pro Glu Gly Phe Ala Gly Pro Asn Cys Ser Ser Val Val Glu
    50                  55                  60

Val Ala Ser Asp Glu Glu Lys Pro Thr Ser Ala Gly Pro Cys Ile Pro
65                  70                  75                  80

Asn Pro Cys His Asn Gly Gly Thr Cys Glu Ile Ser Glu Ala Tyr Arg
                85                  90                  95

Gly Asp Thr Phe Ile Gly Tyr Val Cys Lys Cys Pro Arg Gly Phe Asn
            100                 105                 110

Gly Ile His Cys Gln His Asn Ile Asn Glu Cys Glu Ala Glu Pro Cys
        115                 120                 125

Arg Asn Gly Gly Ile Cys Thr Asp Leu Val Ala Asn Tyr Ser Cys Glu
    130                 135                 140

Cys Pro Gly Glu Phe Met Gly Arg Asn Cys Gln Tyr Lys Cys Ser Gly
145                 150                 155                 160

Pro Leu Gly Ile Glu Gly Gly Ile Ile Ser Asn Gln Gln Ile Thr Ala
                165                 170                 175

Ser Ser Thr His Arg Ala Leu Phe Gly Leu Arg Lys Trp Tyr Pro Tyr
            180                 185                 190

Tyr Ala Arg Leu Asn Lys Lys Gly Leu Ile Asn Ala Trp Thr Ala Ala
        195                 200                 205

Glu Asn Asp Arg Trp Pro Trp Ile Gln Ile Asn Leu Gln Arg Lys Met
    210                 215                 220

Arg Val Thr Gly Val Ile Thr Gln Gly Ala Lys Arg Ile Gly Ser Pro
225                 230                 235                 240

Glu Tyr Ile Lys Ser Tyr Lys Ile Ala Tyr Ser Asn Asp Gly Lys Thr
                245                 250                 255

Trp Ala Met Tyr Lys Val Lys Gly Thr Asn Glu Glu Met Val Phe Arg
            260                 265                 270

Gly Asn Val Asp Asn Asn Thr Pro Tyr Ala Asn Ser Phe Thr Pro Pro
        275                 280                 285

Ile Lys Ala Gln Tyr Val Arg Leu Tyr Pro Gln Ile Cys Arg Arg His
    290                 295                 300

Cys Thr Leu Arg Met Glu Leu Leu Gly Cys Glu Leu Ser Gly Cys Ser
305                 310                 315                 320

Glu Pro Leu Gly Met Lys Ser Gly His Ile Gln Asp Tyr Gln Ile Thr
                325                 330                 335

Ala Ser Ser Val Phe Arg Thr Leu Asn Met Asp Met
            340                 345

<210> 15
<211> 1095
<212> DNA
<213> Mus musculus

<220>
<221> CDS
<222> (1)..(1095)

<400> 15
atg aag cac ttg gta gca gcc tgg ctt ttg gtt gga ctc agc ctc ggg 48
Met Lys His Leu Val Ala Ala Trp Leu Leu Val Gly Leu Ser Leu Gly
1               5                   10                  15

gtg ccc cag ttc ggc aaa ggt gac att tgc aac ccg aac ccc tgt gaa 96
Val Pro Gln Phe Gly Lys Gly Asp Ile Cys Asn Pro Asn Pro Cys Glu
            20                  25                  30

aat ggt ggc atc tgt ctg tca gga ctg gct gat gat tcc ttt tcc tgt 144
Asn Gly Gly Ile Cys Leu Ser Gly Leu Ala Asp Asp Ser Phe Ser Cys
        35                  40                  45

gag tgt cca gaa ggc ttc gca ggt ccg aac tgc tct agt gtt gtg gag 192
Glu Cys Pro Glu Gly Phe Ala Gly Pro Asn Cys Ser Ser Val Val Glu
    50                  55                  60

gtt gca tca gat gaa gaa aag cct act tca gca ggt ccc tgc atc cct 240
Val Ala Ser Asp Glu Glu Lys Pro Thr Ser Ala Gly Pro Cys Ile Pro
65                  70                  75                  80

aac cca tgc cat aac gga gga acc tgt gag ata agc gaa gcc tat cga 288
Asn Pro Cys His Asn Gly Gly Thr Cys Glu Ile Ser Glu Ala Tyr Arg
                85                  90                  95

gga gac aca ttc ata ggc tat gtt tgt aaa tgt cct cgg gga ttt aat 336
Gly Asp Thr Phe Ile Gly Tyr Val Cys Lys Cys Pro Arg Gly Phe Asn
            100                 105                 110

ggg att cac tgt cag cac aat ata aat gaa tgt gaa gct gag cct tgc 384
Gly Ile His Cys Gln His Asn Ile Asn Glu Cys Glu Ala Glu Pro Cys
        115                 120                 125

aga aat ggc gga ata tgt acc gac ctt gtt gct aac tac tct tgt gaa 432
Arg Asn Gly Gly Ile Cys Thr Asp Leu Val Ala Asn Tyr Ser Cys Glu
    130                 135                 140

tgc cca gga gaa ttt atg gga cga aat tgt caa tat aaa tgc tct ggg 480
Cys Pro Gly Glu Phe Met Gly Arg Asn Cys Gln Tyr Lys Cys Ser Gly
145                 150                 155                 160

cca ttg gga atc gaa ggt ggg atc ata tct aat cag caa atc aca gct 528
Pro Leu Gly Ile Glu Gly Gly Ile Ile Ser Asn Gln Gln Ile Thr Ala
                165                 170                 175

tca tct act cac cga gct ctt ttt gga ctc cgg aag tgg tat ccc tac 576
Ser Ser Thr His Arg Ala Leu Phe Gly Leu Arg Lys Trp Tyr Pro Tyr
            180                 185                 190

tat gct cga ctt aat aag aag ggc ctt ata aat gcc tgg aca gct gct 624
Tyr Ala Arg Leu Asn Lys Lys Gly Leu Ile Asn Ala Trp Thr Ala Ala
        195                 200                 205

gaa aat gac aga tgg cca tgg att cag ata aat ttg caa aga aaa atg 672
Glu Asn Asp Arg Trp Pro Trp Ile Gln Ile Asn Leu Gln Arg Lys Met
    210                 215                 220

aga gtc act ggt gtt att acc caa gga gca aaa agg att gga agc cca 720
Arg Val Thr Gly Val Ile Thr Gln Gly Ala Lys Arg Ile Gly Ser Pro
225                 230                 235                 240

gag tac ata aaa tcc tac aaa att gcc tac agc aat gac ggg aag acc 768
Glu Tyr Ile Lys Ser Tyr Lys Ile Ala Tyr Ser Asn Asp Gly Lys Thr
                245                 250                 255

tgg gca atg tac aaa gta aaa ggc acc aat gaa gag atg gtc ttt cgt 816
Trp Ala Met Tyr Lys Val Lys Gly Thr Asn Glu Glu Met Val Phe Arg
            260                 265                 270

gga aat gtt gat aac aac aca cca tat gct aat tct ttc aca ccc cca 864
Gly Asn Val Asp Asn Asn Thr Pro Tyr Ala Asn Ser Phe Thr Pro Pro
        275                 280                 285

atc aaa gct cag tat gta aga ctc tac ccc caa att tgt cga agg cat 912
Ile Lys Ala Gln Tyr Val Arg Leu Tyr Pro Gln Ile Cys Arg Arg His
    290                 295                 300

tgt act tta aga atg gaa ctt ctt ggc tgt gag ctc tca ggc tgt tca     960
Cys Thr Leu Arg Met Glu Leu Leu Gly Cys Glu Leu Ser Gly Cys Ser
305                 310                 315                 320

gaa cct ttg ggg atg aaa tca ggg cat ata caa gac tac cag atc act     1008
Glu Pro Leu Gly Met Lys Ser Gly His Ile Gln Asp Tyr Gln Ile Thr
                325                 330                 335

gcc tcc agc gtc ttc aga aca ctc aac atg gac atg ttt act tgg gaa     1056
Ala Ser Ser Val Phe Arg Thr Leu Asn Met Asp Met Phe Thr Trp Glu
            340                 345                 350

cca agg aaa gcc agg ctg gac aag caa ggc aaa gta aat                 1095
Pro Arg Lys Ala Arg Leu Asp Lys Gln Gly Lys Val Asn
        355                 360                 365

<210>  16
<211>  365
<212>  PRT
<213>  Mus musculus

<400>  16

Met Lys His Leu Val Ala Ala Trp Leu Leu Val Gly Leu Ser Leu Gly
1               5                   10                  15

Val Pro Gln Phe Gly Lys Gly Asp Ile Cys Asn Pro Asn Pro Cys Glu
            20                  25                  30

Asn Gly Gly Ile Cys Leu Ser Gly Leu Ala Asp Asp Ser Phe Ser Cys
        35                  40                  45

Glu Cys Pro Glu Gly Phe Ala Gly Pro Asn Cys Ser Ser Val Val Glu
    50                  55                  60

Val Ala Ser Asp Glu Glu Lys Pro Thr Ser Ala Gly Pro Cys Ile Pro
65                  70                  75                  80

Asn Pro Cys His Asn Gly Gly Thr Cys Glu Ile Ser Glu Ala Tyr Arg
                85                  90                  95

Gly Asp Thr Phe Ile Gly Tyr Val Cys Lys Cys Pro Arg Gly Phe Asn
            100                 105                 110

Gly Ile His Cys Gln His Asn Ile Asn Glu Cys Glu Ala Glu Pro Cys
        115                 120                 125

Arg Asn Gly Gly Ile Cys Thr Asp Leu Val Ala Asn Tyr Ser Cys Glu
    130                 135                 140

Cys Pro Gly Glu Phe Met Gly Arg Asn Cys Gln Tyr Lys Cys Ser Gly
145                 150                 155                 160

Pro Leu Gly Ile Glu Gly Gly Ile Ile Ser Asn Gln Gln Ile Thr Ala
                165                 170                 175

Ser Ser Thr His Arg Ala Leu Phe Gly Leu Arg Lys Trp Tyr Pro Tyr
            180                 185                 190

Tyr Ala Arg Leu Asn Lys Lys Gly Leu Ile Asn Ala Trp Thr Ala Ala
        195                 200                 205

Glu Asn Asp Arg Trp Pro Trp Ile Gln Ile Asn Leu Gln Arg Lys Met
    210                 215                 220

Arg Val Thr Gly Val Ile Thr Gln Gly Ala Lys Arg Ile Gly Ser Pro
225                 230                 235                 240

Glu Tyr Ile Lys Ser Tyr Lys Ile Ala Tyr Ser Asn Asp Gly Lys Thr
                245                 250                 255

Trp Ala Met Tyr Lys Val Lys Gly Thr Asn Glu Glu Met Val Phe Arg
            260                 265                 270

Gly Asn Val Asp Asn Asn Thr Pro Tyr Ala Asn Ser Phe Thr Pro Pro
        275                 280                 285

Ile Lys Ala Gln Tyr Val Arg Leu Tyr Pro Gln Ile Cys Arg Arg His
    290                 295                 300

Cys Thr Leu Arg Met Glu Leu Leu Gly Cys Glu Leu Ser Gly Cys Ser
305                 310                 315                 320

Glu Pro Leu Gly Met Lys Ser Gly His Ile Gln Asp Tyr Gln Ile Thr
                325                 330                 335

Ala Ser Ser Val Phe Arg Thr Leu Asn Met Asp Met Phe Thr Trp Glu
            340                 345                 350

Pro Arg Lys Ala Arg Leu Asp Lys Gln Gly Lys Val Asn
        355                 360                 365

<210>  17
<211>  1104
<212>  DNA
<213>  Mus musculus

<220>
<221>  CDS
<222>  (1)..(1104)

<400>  17
atg aag cac ttg gta gca gcc tgg ctt ttg gtt gga ctc agc ctc ggg        48
Met Lys His Leu Val Ala Ala Trp Leu Leu Val Gly Leu Ser Leu Gly
1               5                   10                  15

gtg ccc cag ttc ggc aaa ggt gac att tgc aac ccg aac ccc tgt gaa        96

Val Pro Gln Phe Gly Lys Gly Asp Ile Cys Asn Pro Asn Pro Cys Glu
            20                  25                  30

aat ggt ggc atc tgt ctg tca gga ctg gct gat gat tcc ttt tcc tgt 144
Asn Gly Gly Ile Cys Leu Ser Gly Leu Ala Asp Asp Ser Phe Ser Cys
        35                  40                  45

gag tgt cca gaa ggc ttc gca ggt ccg aac tgc tct agt gtt gtg gag 192
Glu Cys Pro Glu Gly Phe Ala Gly Pro Asn Cys Ser Ser Val Val Glu
    50                  55                  60

gtt gca tca gat gaa gaa aag cct act tca gca ggt ccc tgc atc cct 240
Val Ala Ser Asp Glu Glu Lys Pro Thr Ser Ala Gly Pro Cys Ile Pro
65                  70                  75                  80

aac cca tgc cat aac gga gga acc tgt gag ata agc gaa gcc tat cga 288
Asn Pro Cys His Asn Gly Gly Thr Cys Glu Ile Ser Glu Ala Tyr Arg
                85                  90                  95

gga gac aca ttc ata ggc tat gtt tgt aaa tgt cct cgg gga ttt aat 336
Gly Asp Thr Phe Ile Gly Tyr Val Cys Lys Cys Pro Arg Gly Phe Asn
            100                 105                 110

ggg att cac tgt cag cac aat ata aat gaa tgt gaa gct gag cct tgc 384
Gly Ile His Cys Gln His Asn Ile Asn Glu Cys Glu Ala Glu Pro Cys
        115                 120                 125

aga aat ggc gga ata tgt acc gac ctt gtt gct aac tac tct tgt gaa 432
Arg Asn Gly Gly Ile Cys Thr Asp Leu Val Ala Asn Tyr Ser Cys Glu
    130                 135                 140

tgc cca gga gaa ttt atg gga cga aat tgt caa tat aaa tgc tct ggg 480
Cys Pro Gly Glu Phe Met Gly Arg Asn Cys Gln Tyr Lys Cys Ser Gly
145                 150                 155                 160

cca ttg gga atc gaa ggt ggg atc ata tct aat cag caa atc aca gct 528
Pro Leu Gly Ile Glu Gly Gly Ile Ile Ser Asn Gln Gln Ile Thr Ala
                165                 170                 175

tca tct act cac cga gct ctt ttt gga ctc cgg aag tgg tat ccc tac 576
Ser Ser Thr His Arg Ala Leu Phe Gly Leu Arg Lys Trp Tyr Pro Tyr
            180                 185                 190

tat gct cga ctt aat aag aag ggc ctt ata aat gcc tgg aca gct gct 624

Tyr Ala Arg Leu Asn Lys Lys Gly Leu Ile Asn Ala Trp Thr Ala Ala
        195                 200                 205

gaa aat gac aga tgg cca tgg att cag ata aat ttg caa aga aaa atg     672
Glu Asn Asp Arg Trp Pro Trp Ile Gln Ile Asn Leu Gln Arg Lys Met
    210                 215                 220

aga gtc act ggt gtt att acc caa gga gca aaa agg att gga agc cca     720
Arg Val Thr Gly Val Ile Thr Gln Gly Ala Lys Arg Ile Gly Ser Pro
225                 230                 235                 240

gag tac ata aaa tcc tac aaa att gcc tac agc aat gac ggg aag acc     768
Glu Tyr Ile Lys Ser Tyr Lys Ile Ala Tyr Ser Asn Asp Gly Lys Thr
                245                 250                 255

tgg gca atg tac aaa gta aaa ggc acc aat gaa gag atg gtc ttt cgt     816
Trp Ala Met Tyr Lys Val Lys Gly Thr Asn Glu Glu Met Val Phe Arg
            260                 265                 270

gga aat gtt gat aac aac aca cca tat gct aat tct ttc aca ccc cca     864
Gly Asn Val Asp Asn Asn Thr Pro Tyr Ala Asn Ser Phe Thr Pro Pro
        275                 280                 285

atc aaa gct cag tat gta aga ctc tac ccc caa att tgt cga agg cat     912
Ile Lys Ala Gln Tyr Val Arg Leu Tyr Pro Gln Ile Cys Arg Arg His
    290                 295                 300

tgt act tta aga atg gaa ctt ctt ggc tgt gag ctc tca ggc tgt tca     960
Cys Thr Leu Arg Met Glu Leu Leu Gly Cys Glu Leu Ser Gly Cys Ser
305                 310                 315                 320

gaa cct ttg ggg atg aaa tca ggg cat ata caa gac tac cag atc act     1008
Glu Pro Leu Gly Met Lys Ser Gly His Ile Gln Asp Tyr Gln Ile Thr
                325                 330                 335

gcc tcc agc gtc ttc aga aca ctc aac atg gac atg ttt act tgg gaa     1056
Ala Ser Ser Val Phe Arg Thr Leu Asn Met Asp Met Phe Thr Trp Glu
            340                 345                 350

cca agg aaa gcc agg ctg gac aag caa ggc aaa gta aat gcc tgg act     1104
Pro Arg Lys Ala Arg Leu Asp Lys Gln Gly Lys Val Asn Ala Trp Thr
        355                 360                 365

<210> 18
<211> 368
<212> PRT
<213> Mus musculus

<400> 18

Met Lys His Leu Val Ala Ala Trp Leu Leu Val Gly Leu Ser Leu Gly
1               5                   10                  15

Val Pro Gln Phe Gly Lys Gly Asp Ile Cys Asn Pro Asn Pro Cys Glu
            20                  25                  30

Asn Gly Gly Ile Cys Leu Ser Gly Leu Ala Asp Asp Ser Phe Ser Cys
        35                  40                  45

Glu Cys Pro Glu Gly Phe Ala Gly Pro Asn Cys Ser Ser Val Val Glu
    50                  55                  60

Val Ala Ser Asp Glu Glu Lys Pro Thr Ser Ala Gly Pro Cys Ile Pro
65                  70                  75                  80

Asn Pro Cys His Asn Gly Gly Thr Cys Glu Ile Ser Glu Ala Tyr Arg
                85                  90                  95

Gly Asp Thr Phe Ile Gly Tyr Val Cys Lys Cys Pro Arg Gly Phe Asn
            100                 105                 110

Gly Ile His Cys Gln His Asn Ile Asn Glu Cys Glu Ala Glu Pro Cys
        115                 120                 125

Arg Asn Gly Gly Ile Cys Thr Asp Leu Val Ala Asn Tyr Ser Cys Glu
    130                 135                 140

Cys Pro Gly Glu Phe Met Gly Arg Asn Cys Gln Tyr Lys Cys Ser Gly

145                 150                 155                 160

Pro Leu Gly Ile Glu Gly Gly Ile Ile Ser Asn Gln Gln Ile Thr Ala
                165                 170                 175

Ser Ser Thr His Arg Ala Leu Phe Gly Leu Arg Lys Trp Tyr Pro Tyr
            180                 185                 190

Tyr Ala Arg Leu Asn Lys Lys Gly Leu Ile Asn Ala Trp Thr Ala Ala
        195                 200                 205

Glu Asn Asp Arg Trp Pro Trp Ile Gln Ile Asn Leu Gln Arg Lys Met
    210                 215                 220

Arg Val Thr Gly Val Ile Thr Gln Gly Ala Lys Arg Ile Gly Ser Pro
225                 230                 235                 240

Glu Tyr Ile Lys Ser Tyr Lys Ile Ala Tyr Ser Asn Asp Gly Lys Thr
                245                 250                 255

Trp Ala Met Tyr Lys Val Lys Gly Thr Asn Glu Glu Met Val Phe Arg
            260                 265                 270

Gly Asn Val Asp Asn Asn Thr Pro Tyr Ala Asn Ser Phe Thr Pro Pro
        275                 280                 285

Ile Lys Ala Gln Tyr Val Arg Leu Tyr Pro Gln Ile Cys Arg Arg His
    290                 295                 300

Cys Thr Leu Arg Met Glu Leu Leu Gly Cys Glu Leu Ser Gly Cys Ser
305                 310                 315                 320

Glu Pro Leu Gly Met Lys Ser Gly His Ile Gln Asp Tyr Gln Ile Thr

                325                 330                 335

Ala Ser Ser Val Phe Arg Thr Leu Asn Met Asp Met Phe Thr Trp Glu
            340                 345                 350

Pro Arg Lys Ala Arg Leu Asp Lys Gln Gly Lys Val Asn Ala Trp Thr
        355                 360                 365

<210> 19
<211> 1155
<212> DNA
<213> Mus musculus

<220>
<221> CDS
<222> (1)..(1155)

<400> 19
atg aag cac ttg gta gca gcc tgg ctt ttg gtt gga ctc agc ctc ggg 48
Met Lys His Leu Val Ala Ala Trp Leu Leu Val Gly Leu Ser Leu Gly
1               5                   10                  15

gtg ccc cag ttc ggc aaa ggt gac att tgc aac ccg aac ccc tgt gaa 96
Val Pro Gln Phe Gly Lys Gly Asp Ile Cys Asn Pro Asn Pro Cys Glu
            20                  25                  30

aat ggt ggc atc tgt ctg tca gga ctg gct gat gat tcc ttt tcc tgt 144
Asn Gly Gly Ile Cys Leu Ser Gly Leu Ala Asp Asp Ser Phe Ser Cys
        35                  40                  45

gag tgt cca gaa ggc ttc gca ggt ccg aac tgc tct agt gtt gtg gag 192
Glu Cys Pro Glu Gly Phe Ala Gly Pro Asn Cys Ser Ser Val Val Glu
    50                  55                  60

gtt gca tca gat gaa gaa aag cct act tca gca ggt ccc tgc atc cct 240
Val Ala Ser Asp Glu Glu Lys Pro Thr Ser Ala Gly Pro Cys Ile Pro
65                  70                  75                  80

aac cca tgc cat aac gga gga acc tgt gag ata agc gaa gcc tat cga 288
Asn Pro Cys His Asn Gly Gly Thr Cys Glu Ile Ser Glu Ala Tyr Arg

85                  90                  95

gga gac aca ttc ata ggc tat gtt tgt aaa tgt cct cgg gga ttt aat      336
Gly Asp Thr Phe Ile Gly Tyr Val Cys Lys Cys Pro Arg Gly Phe Asn
            100                 105                 110

ggg att cac tgt cag cac aat ata aat gaa tgt gaa gct gag cct tgc      384
Gly Ile His Cys Gln His Asn Ile Asn Glu Cys Glu Ala Glu Pro Cys
        115                 120                 125

aga aat ggc gga ata tgt acc gac ctt gtt gct aac tac tct tgt gaa      432
Arg Asn Gly Gly Ile Cys Thr Asp Leu Val Ala Asn Tyr Ser Cys Glu
    130                 135                 140

tgc cca gga gaa ttt atg gga cga aat tgt caa tat aaa tgc tct ggg      480
Cys Pro Gly Glu Phe Met Gly Arg Asn Cys Gln Tyr Lys Cys Ser Gly
145                 150                 155                 160

cca ttg gga atc gaa ggt ggg atc ata tct aat cag caa atc aca gct      528
Pro Leu Gly Ile Glu Gly Gly Ile Ile Ser Asn Gln Gln Ile Thr Ala
                165                 170                 175

tca tct act cac cga gct ctt ttt gga ctc cgg aag tgg tat ccc tac      576
Ser Ser Thr His Arg Ala Leu Phe Gly Leu Arg Lys Trp Tyr Pro Tyr
            180                 185                 190

tat gct cga ctt aat aag aag ggc ctt ata aat gcc tgg aca gct gct      624
Tyr Ala Arg Leu Asn Lys Lys Gly Leu Ile Asn Ala Trp Thr Ala Ala
        195                 200                 205

gaa aat gac aga tgg cca tgg att cag ata aat ttg caa aga aaa atg      672
Glu Asn Asp Arg Trp Pro Trp Ile Gln Ile Asn Leu Gln Arg Lys Met
    210                 215                 220

aga gtc act ggt gtt att acc caa gga gca aaa agg att gga agc cca      720
Arg Val Thr Gly Val Ile Thr Gln Gly Ala Lys Arg Ile Gly Ser Pro
225                 230                 235                 240

gag tac ata aaa tcc tac aaa att gcc tac agc aat gac ggg aag acc      768
Glu Tyr Ile Lys Ser Tyr Lys Ile Ala Tyr Ser Asn Asp Gly Lys Thr
                245                 250                 255

tgg gca atg tac aaa gta aaa ggc acc aat gaa gag atg gtc ttt cgt      816
Trp Ala Met Tyr Lys Val Lys Gly Thr Asn Glu Glu Met Val Phe Arg

            260                 265                 270

gga aat gtt gat aac aac aca cca tat gct aat tct ttc aca ccc cca 864
Gly Asn Val Asp Asn Asn Thr Pro Tyr Ala Asn Ser Phe Thr Pro Pro
        275                 280                 285

atc aaa gct cag tat gta aga ctc tac ccc caa att tgt cga agg cat 912
Ile Lys Ala Gln Tyr Val Arg Leu Tyr Pro Gln Ile Cys Arg Arg His
    290                 295                 300

tgt act tta aga atg gaa ctt ctt ggc tgt gag ctc tca ggc tgt tca 960
Cys Thr Leu Arg Met Glu Leu Leu Gly Cys Glu Leu Ser Gly Cys Ser
305                 310                 315                 320

gaa cct ttg ggg atg aaa tca ggg cat ata caa gac tac cag atc act 1008
Glu Pro Leu Gly Met Lys Ser Gly His Ile Gln Asp Tyr Gln Ile Thr
                325                 330                 335

gcc tcc agc gtc ttc aga aca ctc aac atg gac atg ttt act tgg gaa 1056
Ala Ser Ser Val Phe Arg Thr Leu Asn Met Asp Met Phe Thr Trp Glu
            340                 345                 350

cca agg aaa gcc agg ctg gac aag caa ggc aaa gta aat gcc tgg act 1104
Pro Arg Lys Ala Arg Leu Asp Lys Gln Gly Lys Val Asn Ala Trp Thr
        355                 360                 365

tcc ggc cat aac gac cag tca caa tgg tta cag gtt gat ctt ctt gtc 1152
Ser Gly His Asn Asp Gln Ser Gln Trp Leu Gln Val Asp Leu Leu Val
    370                 375                 380

cct 1155
Pro
385

<210> 20
<211> 385
<212> PRT
<213> Mus musculus

<400> 20

Met Lys His Leu Val Ala Ala Trp Leu Leu Val Gly Leu Ser Leu Gly
1               5                   10                  15

Val Pro Gln Phe Gly Lys Gly Asp Ile Cys Asn Pro Asn Pro Cys Glu
            20                  25                  30

Asn Gly Gly Ile Cys Leu Ser Gly Leu Ala Asp Asp Ser Phe Ser Cys
        35                  40                  45

Glu Cys Pro Glu Gly Phe Ala Gly Pro Asn Cys Ser Ser Val Val Glu
    50                  55                  60

Val Ala Ser Asp Glu Glu Lys Pro Thr Ser Ala Gly Pro Cys Ile Pro
65                  70                  75                  80

Asn Pro Cys His Asn Gly Gly Thr Cys Glu Ile Ser Glu Ala Tyr Arg
                85                  90                  95

Gly Asp Thr Phe Ile Gly Tyr Val Cys Lys Cys Pro Arg Gly Phe Asn
            100                 105                 110

Gly Ile His Cys Gln His Asn Ile Asn Glu Cys Glu Ala Glu Pro Cys
        115                 120                 125

Arg Asn Gly Gly Ile Cys Thr Asp Leu Val Ala Asn Tyr Ser Cys Glu
    130                 135                 140

Cys Pro Gly Glu Phe Met Gly Arg Asn Cys Gln Tyr Lys Cys Ser Gly
145                 150                 155                 160

Pro Leu Gly Ile Glu Gly Gly Ile Ile Ser Asn Gln Gln Ile Thr Ala
                165                 170                 175

Ser Ser Thr His Arg Ala Leu Phe Gly Leu Arg Lys Trp Tyr Pro Tyr
            180                 185                 190

Tyr Ala Arg Leu Asn Lys Lys Gly Leu Ile Asn Ala Trp Thr Ala Ala
        195                 200                 205

Glu Asn Asp Arg Trp Pro Trp Ile Gln Ile Asn Leu Gln Arg Lys Met
    210                 215                 220

Arg Val Thr Gly Val Ile Thr Gln Gly Ala Lys Arg Ile Gly Ser Pro
225                 230                 235                 240

Glu Tyr Ile Lys Ser Tyr Lys Ile Ala Tyr Ser Asn Asp Gly Lys Thr
                245                 250                 255

Trp Ala Met Tyr Lys Val Lys Gly Thr Asn Glu Glu Met Val Phe Arg
            260                 265                 270

Gly Asn Val Asp Asn Asn Thr Pro Tyr Ala Asn Ser Phe Thr Pro Pro
        275                 280                 285

Ile Lys Ala Gln Tyr Val Arg Leu Tyr Pro Gln Ile Cys Arg Arg His
    290                 295                 300

Cys Thr Leu Arg Met Glu Leu Leu Gly Cys Glu Leu Ser Gly Cys Ser
305                 310                 315                 320

Glu Pro Leu Gly Met Lys Ser Gly His Ile Gln Asp Tyr Gln Ile Thr
                325                 330                 335

Ala Ser Ser Val Phe Arg Thr Leu Asn Met Asp Met Phe Thr Trp Glu
            340                 345                 350

Pro Arg Lys Ala Arg Leu Asp Lys Gln Gly Lys Val Asn Ala Trp Thr
        355                 360                 365

Ser Gly His Asn Asp Gln Ser Gln Trp Leu Gln Val Asp Leu Leu Val
    370                 375                 380

Pro
385

<210>  21
<211>  678
<212>  DNA
<213>  Mus musculus

<220>
<221>  CDS
<222>  (1)..(678)

<400>  21
tgt gaa gct gag cct tgc aga aat ggc gga ata tgt acc gac ctt gtt      48
Cys Glu Ala Glu Pro Cys Arg Asn Gly Gly Ile Cys Thr Asp Leu Val
1               5                   10                  15

gct aac tac tct tgt gaa tgc cca gga gaa ttt atg gga cga aat tgt      96
Ala Asn Tyr Ser Cys Glu Cys Pro Gly Glu Phe Met Gly Arg Asn Cys
            20                  25                  30

caa tat aaa tgc tct ggg cca ttg gga atc gaa ggt ggg atc ata tct      144
Gln Tyr Lys Cys Ser Gly Pro Leu Gly Ile Glu Gly Gly Ile Ile Ser
        35                  40                  45

aat cag caa atc aca gct tca tct act cac cga gct ctt ttt gga ctc      192
Asn Gln Gln Ile Thr Ala Ser Ser Thr His Arg Ala Leu Phe Gly Leu
    50                  55                  60

cgg aag tgg tat ccc tac tat gct cga ctt aat aag aag ggc ctt ata      240
Arg Lys Trp Tyr Pro Tyr Tyr Ala Arg Leu Asn Lys Lys Gly Leu Ile
65                  70                  75                  80

aat gcc tgg aca gct gct gaa aat gac aga tgg cca tgg att cag ata      288
Asn Ala Trp Thr Ala Ala Glu Asn Asp Arg Trp Pro Trp Ile Gln Ile
                85                  90                  95

aat ttg caa aga aaa atg aga gtc act ggt gtt att acc caa gga gca 336
Asn Leu Gln Arg Lys Met Arg Val Thr Gly Val Ile Thr Gln Gly Ala
            100                 105                 110

aaa agg att gga agc cca gag tac ata aaa tcc tac aaa att gcc tac 384
Lys Arg Ile Gly Ser Pro Glu Tyr Ile Lys Ser Tyr Lys Ile Ala Tyr
        115                 120                 125

agc aat gac ggg aag acc tgg gca atg tac aaa gta aaa ggc acc aat 432
Ser Asn Asp Gly Lys Thr Trp Ala Met Tyr Lys Val Lys Gly Thr Asn
    130                 135                 140

gaa gag atg gtc ttt cgt gga aat gtt gat aac aac aca cca tat gct 480
Glu Glu Met Val Phe Arg Gly Asn Val Asp Asn Asn Thr Pro Tyr Ala
145                 150                 155                 160

aat tct ttc aca ccc cca atc aaa gct cag tat gta aga ctc tac ccc 528
Asn Ser Phe Thr Pro Pro Ile Lys Ala Gln Tyr Val Arg Leu Tyr Pro
                165                 170                 175

caa att tgt cga agg cat tgt act tta aga atg gaa ctt ctt ggc tgt 576
Gln Ile Cys Arg Arg His Cys Thr Leu Arg Met Glu Leu Leu Gly Cys
            180                 185                 190

gag ctc tca ggc tgt tca gaa cct ttg ggg atg aaa tca ggg cat ata 624
Glu Leu Ser Gly Cys Ser Glu Pro Leu Gly Met Lys Ser Gly His Ile
        195                 200                 205

caa gac tac cag atc act gcc tcc agc gtc ttc aga aca ctc aac atg 672
Gln Asp Tyr Gln Ile Thr Ala Ser Ser Val Phe Arg Thr Leu Asn Met
    210                 215                 220

gac atg 678
Asp Met
225

<210> 22
<211> 226
<212> PRT
<213> Mus musculus

<400> 22

Cys Glu Ala Glu Pro Cys Arg Asn Gly Gly Ile Cys Thr Asp Leu Val
1               5                   10                  15

Ala Asn Tyr Ser Cys Glu Cys Pro Gly Glu Phe Met Gly Arg Asn Cys
            20                  25                  30

Gln Tyr Lys Cys Ser Gly Pro Leu Gly Ile Glu Gly Gly Ile Ile Ser
        35                  40                  45

Asn Gln Gln Ile Thr Ala Ser Ser Thr His Arg Ala Leu Phe Gly Leu
    50                  55                  60

Arg Lys Trp Tyr Pro Tyr Tyr Ala Arg Leu Asn Lys Lys Gly Leu Ile
65                  70                  75                  80

Asn Ala Trp Thr Ala Ala Glu Asn Asp Arg Trp Pro Trp Ile Gln Ile
                85                  90                  95

Asn Leu Gln Arg Lys Met Arg Val Thr Gly Val Ile Thr Gln Gly Ala
            100                 105                 110

Lys Arg Ile Gly Ser Pro Glu Tyr Ile Lys Ser Tyr Lys Ile Ala Tyr
        115                 120                 125

Ser Asn Asp Gly Lys Thr Trp Ala Met Tyr Lys Val Lys Gly Thr Asn
    130                 135                 140

Glu Glu Met Val Phe Arg Gly Asn Val Asp Asn Asn Thr Pro Tyr Ala
145                 150                 155                 160

Asn Ser Phe Thr Pro Pro Ile Lys Ala Gln Tyr Val Arg Leu Tyr Pro
                165                 170                 175

Gln Ile Cys Arg Arg His Cys Thr Leu Arg Met Glu Leu Leu Gly Cys
            180                 185                 190

Glu Leu Ser Gly Cys Ser Glu Pro Leu Gly Met Lys Ser Gly His Ile
        195                 200                 205

Gln Asp Tyr Gln Ile Thr Ala Ser Ser Val Phe Arg Thr Leu Asn Met
    210                 215                 220

Asp Met
225

<210>  23
<211>  285
<212>  DNA
<213>  Mus musculus

<220>
<221>  CDS
<222>  (1)..(285)

<400>  23
tgt gaa gct gag cct tgc aga aat ggc gga ata tgt acc gac ctt gtt      48
Cys Glu Ala Glu Pro Cys Arg Asn Gly Gly Ile Cys Thr Asp Leu Val
1               5                   10                  15

gct aac tac tct tgt gaa tgc cca gga gaa ttt atg gga cga aat tgt      96
Ala Asn Tyr Ser Cys Glu Cys Pro Gly Glu Phe Met Gly Arg Asn Cys
            20                  25                  30

caa tat aaa tgc tct ggg cca ttg gga atc gaa ggt ggg atc ata tct      144
Gln Tyr Lys Cys Ser Gly Pro Leu Gly Ile Glu Gly Gly Ile Ile Ser
        35                  40                  45

aat cag caa atc aca gct tca tct act cac cga gct ctt ttt gga ctc      192
Asn Gln Gln Ile Thr Ala Ser Ser Thr His Arg Ala Leu Phe Gly Leu
    50                  55                  60

cgg aag tgg tat ccc tac tat gct cga ctt aat aag aag ggc ctt ata     240
Arg Lys Trp Tyr Pro Tyr Tyr Ala Arg Leu Asn Lys Lys Gly Leu Ile
65                  70                  75                  80

aat gcc tgg aca gct gct gaa aat gac aga tgg cca tgg att cag         285
Asn Ala Trp Thr Ala Ala Glu Asn Asp Arg Trp Pro Trp Ile Gln
                85                  90                  95

<210>  24
<211>  95
<212>  PRT
<213>  Mus musculus

<400>  24

Cys Glu Ala Glu Pro Cys Arg Asn Gly Gly Ile Cys Thr Asp Leu Val
1               5                   10                  15

Ala Asn Tyr Ser Cys Glu Cys Pro Gly Glu Phe Met Gly Arg Asn Cys
            20                  25                  30

Gln Tyr Lys Cys Ser Gly Pro Leu Gly Ile Glu Gly Gly Ile Ile Ser
        35                  40                  45

Asn Gln Gln Ile Thr Ala Ser Ser Thr His Arg Ala Leu Phe Gly Leu
    50                  55                  60

Arg Lys Trp Tyr Pro Tyr Tyr Ala Arg Leu Asn Lys Lys Gly Leu Ile
65                  70                  75                  80

Asn Ala Trp Thr Ala Ala Glu Asn Asp Arg Trp Pro Trp Ile Gln
                85                  90                  95

<210>  25
<211>  678
<212>  DNA
<213>  Homo sapiens

<220>
<221> CDS
<222> (1)..(678)

<400> 25
tgc gaa gtt gag cct tgc aaa aat ggt gga ata tgt aca gat ctt gtt 48
Cys Glu Val Glu Pro Cys Lys Asn Gly Gly Ile Cys Thr Asp Leu Val
1               5                   10                  15

gct aac tat tcc tgt gag tgc cca ggc gaa ttt atg gga aga aat tgt 96
Ala Asn Tyr Ser Cys Glu Cys Pro Gly Glu Phe Met Gly Arg Asn Cys
            20                  25                  30

caa tac aaa tgc tca ggc cca ctg gga att gaa ggt gga att ata tca 144
Gln Tyr Lys Cys Ser Gly Pro Leu Gly Ile Glu Gly Gly Ile Ile Ser
        35                  40                  45

aac cag caa atc aca gct tcc tct act cac cga gct ctt ttt gga ctc 192
Asn Gln Gln Ile Thr Ala Ser Ser Thr His Arg Ala Leu Phe Gly Leu
    50                  55                  60

caa aaa tgg tat ccc tac tat gca cgt ctt aat aag aag ggg ctt ata 240
Gln Lys Trp Tyr Pro Tyr Tyr Ala Arg Leu Asn Lys Lys Gly Leu Ile
65                  70                  75                  80

aat gcg tgg aca gct gca gaa aat gac aga tgg ccg tgg att cag ata 288
Asn Ala Trp Thr Ala Ala Glu Asn Asp Arg Trp Pro Trp Ile Gln Ile
                85                  90                  95

aat ttg caa agg aaa atg aga gtt act ggt gtg att acc caa gga gcc 336
Asn Leu Gln Arg Lys Met Arg Val Thr Gly Val Ile Thr Gln Gly Ala
            100                 105                 110

aag agg att gga agc cca gag tat ata aaa tcc tac aaa att gcc tac 384
Lys Arg Ile Gly Ser Pro Glu Tyr Ile Lys Ser Tyr Lys Ile Ala Tyr
        115                 120                 125

agt aat gat gga aag act tgg gca atg tac aaa gtg aaa ggc acc aat 432
Ser Asn Asp Gly Lys Thr Trp Ala Met Tyr Lys Val Lys Gly Thr Asn
    130                 135                 140

gaa gac atg gtg ttt cgt gga aac att gat aac aac act cca tat gct 480

Glu Asp Met Val Phe Arg Gly Asn Ile Asp Asn Asn Thr Pro Tyr Ala
145                 150                 155                 160

aac tct ttc aca ccc ccc ata aaa gct cag tat gta aga ctc tat ccc     528
Asn Ser Phe Thr Pro Pro Ile Lys Ala Gln Tyr Val Arg Leu Tyr Pro
                165                 170                 175

caa gtt tgt cga aga cat tgc act ttg cga atg gaa ctt ctt ggc tgt     576
Gln Val Cys Arg Arg His Cys Thr Leu Arg Met Glu Leu Leu Gly Cys
            180                 185                 190

gaa ctg tcg ggt tgt tct gag cct ctg ggt atg aaa tca gga cat ata     624
Glu Leu Ser Gly Cys Ser Glu Pro Leu Gly Met Lys Ser Gly His Ile
        195                 200                 205

caa gac tat cag atc act gcc tcc agc atc ttc aga acg ctc aac atg     672
Gln Asp Tyr Gln Ile Thr Ala Ser Ser Ile Phe Arg Thr Leu Asn Met
    210                 215                 220

gac atg                                                             678
Asp Met
225

<210>  26
<211>  226
<212>  PRT
<213>  Homo sapiens

<400>  26

Cys Glu Val Glu Pro Cys Lys Asn Gly Gly Ile Cys Thr Asp Leu Val
1               5                   10                  15

Ala Asn Tyr Ser Cys Glu Cys Pro Gly Glu Phe Met Gly Arg Asn Cys
            20                  25                  30

Gln Tyr Lys Cys Ser Gly Pro Leu Gly Ile Glu Gly Gly Ile Ile Ser
        35                  40                  45

Asn Gln Gln Ile Thr Ala Ser Ser Thr His Arg Ala Leu Phe Gly Leu

50                  55                  60

Gln Lys Trp Tyr Pro Tyr Tyr Ala Arg Leu Asn Lys Lys Gly Leu Ile
65                  70                  75                  80

Asn Ala Trp Thr Ala Ala Glu Asn Asp Arg Trp Pro Trp Ile Gln Ile
                85                  90                  95

Asn Leu Gln Arg Lys Met Arg Val Thr Gly Val Ile Thr Gln Gly Ala
            100                 105                 110

Lys Arg Ile Gly Ser Pro Glu Tyr Ile Lys Ser Tyr Lys Ile Ala Tyr
        115                 120                 125

Ser Asn Asp Gly Lys Thr Trp Ala Met Tyr Lys Val Lys Gly Thr Asn
    130                 135                 140

Glu Asp Met Val Phe Arg Gly Asn Ile Asp Asn Asn Thr Pro Tyr Ala
145                 150                 155                 160

Asn Ser Phe Thr Pro Pro Ile Lys Ala Gln Tyr Val Arg Leu Tyr Pro
                165                 170                 175

Gln Val Cys Arg Arg His Cys Thr Leu Arg Met Glu Leu Leu Gly Cys
            180                 185                 190

Glu Leu Ser Gly Cys Ser Glu Pro Leu Gly Met Lys Ser Gly His Ile
        195                 200                 205

Gln Asp Tyr Gln Ile Thr Ala Ser Ser Ile Phe Arg Thr Leu Asn Met
    210                 215                 220

Asp Met

225

<210> 27
<211> 1395
<212> DNA
<213> Mus musculus

<220>
<221> CDS
<222> (1)..(1395)

<400> 27
atg gca gaa tcc ccg gct ctc atc acc atc ttc ctt tta gga tat cta 48
Met Ala Glu Ser Pro Ala Leu Ile Thr Ile Phe Leu Leu Gly Tyr Leu
1               5                   10                  15

ctc agt acc gaa tgt gca gtt ttc ctt gat cgt gaa aat gcc acc aaa 96
Leu Ser Thr Glu Cys Ala Val Phe Leu Asp Arg Glu Asn Ala Thr Lys
            20                  25                  30

att ctt acc cgt cca aag aga tat aat tca gga aaa cta gaa gag ttt 144
Ile Leu Thr Arg Pro Lys Arg Tyr Asn Ser Gly Lys Leu Glu Glu Phe
        35                  40                  45

gtt cga gga aac ctt gaa aga gag tgt ata gaa gaa aga tgt agt ttt 192
Val Arg Gly Asn Leu Glu Arg Glu Cys Ile Glu Glu Arg Cys Ser Phe
    50                  55                  60

gaa gaa gca cga gaa gtt ttt gaa aac act gaa aaa act act gaa ttt 240
Glu Glu Ala Arg Glu Val Phe Glu Asn Thr Glu Lys Thr Thr Glu Phe
65                  70                  75                  80

tgg aag cag tat gtt gat gga gat cag tgt gaa tca aat cct tgt tta 288
Trp Lys Gln Tyr Val Asp Gly Asp Gln Cys Glu Ser Asn Pro Cys Leu
                85                  90                  95

aat ggt gga ata tgc aag gat gat att agt tcc tat gaa tgc tgg tgc 336
Asn Gly Gly Ile Cys Lys Asp Asp Ile Ser Ser Tyr Glu Cys Trp Cys
            100                 105                 110

caa gtt gga ttt gaa gga agg aac tgt gaa tta gat gca acg tgt aac 384
Gln Val Gly Phe Glu Gly Arg Asn Cys Glu Leu Asp Ala Thr Cys Asn

        115                 120                 125

att aaa aat ggc agg tgc aag cag ttt tgt aaa aac agt cct gat aac 432
Ile Lys Asn Gly Arg Cys Lys Gln Phe Cys Lys Asn Ser Pro Asp Asn
    130                 135                 140

aag gta att tgt tcc tgc act gag gga tac caa ctt gca gaa gac cag 480
Lys Val Ile Cys Ser Cys Thr Glu Gly Tyr Gln Leu Ala Glu Asp Gln
145                 150                 155                 160

aag tcc tgt gaa cca aca gtt cca ttt cca tgt ggg aga gct tct att 528
Lys Ser Cys Glu Pro Thr Val Pro Phe Pro Cys Gly Arg Ala Ser Ile
                165                 170                 175

tca tac agt tct aaa aag atc acg aga gct gag act gtt ttc tct aat 576
Ser Tyr Ser Ser Lys Lys Ile Thr Arg Ala Glu Thr Val Phe Ser Asn
            180                 185                 190

atg gac tat gaa aat tct act gaa gct gta ttc att caa gat gac atc 624
Met Asp Tyr Glu Asn Ser Thr Glu Ala Val Phe Ile Gln Asp Asp Ile
        195                 200                 205

act gat ggt gcc att ctt aat aac gtc act gaa agt agt gaa tca ctt 672
Thr Asp Gly Ala Ile Leu Asn Asn Val Thr Glu Ser Ser Glu Ser Leu
    210                 215                 220

aat gac ttc act cga gtt gtt ggt gga gaa aac gca aaa ccg ggt caa 720
Asn Asp Phe Thr Arg Val Val Gly Gly Glu Asn Ala Lys Pro Gly Gln
225                 230                 235                 240

atc cct tgg cag gtc att tta aat ggt gaa att gag gca ttc tgt gga 768
Ile Pro Trp Gln Val Ile Leu Asn Gly Glu Ile Glu Ala Phe Cys Gly
                245                 250                 255

ggt gcc atc att aat gaa aaa tgg att gta act gct gcc cac tgt ctt 816
Gly Ala Ile Ile Asn Glu Lys Trp Ile Val Thr Ala Ala His Cys Leu
            260                 265                 270

aaa cct ggt gat aaa att gag gtt gtt gct ggt gaa tat aac att gat 864
Lys Pro Gly Asp Lys Ile Glu Val Val Ala Gly Glu Tyr Asn Ile Asp
        275                 280                 285

aag aag gaa gac aca gaa caa agg aga aat gtg att cga act atc cct 912
Lys Lys Glu Asp Thr Glu Gln Arg Arg Asn Val Ile Arg Thr Ile Pro

290                 295                 300

cat cac cag tac aat gca act att aat aag tat agt cat gac att gcc        960
His His Gln Tyr Asn Ala Thr Ile Asn Lys Tyr Ser His Asp Ile Ala
305                 310                 315                 320

ttg ctg gaa ctg gat aaa cct tta ata cta aac agc tat gta aca cct        1008
Leu Leu Glu Leu Asp Lys Pro Leu Ile Leu Asn Ser Tyr Val Thr Pro
                325                 330                 335

atc tgt gtt gcc aat agg gaa tat aca aat atc ttc ctc aag ttt ggt        1056
Ile Cys Val Ala Asn Arg Glu Tyr Thr Asn Ile Phe Leu Lys Phe Gly
            340                 345                 350

tct ggc tat gtc agt ggc tgg gga aaa gtc ttc aac aaa ggg aga cag        1104
Ser Gly Tyr Val Ser Gly Trp Gly Lys Val Phe Asn Lys Gly Arg Gln
        355                 360                 365

gct tcc att ctt cag tac ctt aga gtt cca ctg gtg gat aga gcc aca        1152
Ala Ser Ile Leu Gln Tyr Leu Arg Val Pro Leu Val Asp Arg Ala Thr
    370                 375                 380

tgc ctt agg tcc aca aca ttc act atc tat aac aac atg ttc tgt gca        1200
Cys Leu Arg Ser Thr Thr Phe Thr Ile Tyr Asn Asn Met Phe Cys Ala
385                 390                 395                 400

ggc tac cgt gaa gga ggc aaa gat tcg tgt gaa gga gat agt ggg gga        1248
Gly Tyr Arg Glu Gly Gly Lys Asp Ser Cys Glu Gly Asp Ser Gly Gly
                405                 410                 415

ccc cat gtt act gaa gta gaa ggg aca agt ttc tta act ggc att att        1296
Pro His Val Thr Glu Val Glu Gly Thr Ser Phe Leu Thr Gly Ile Ile
            420                 425                 430

agc tgg ggt gaa gaa tgt gca atg aaa ggc aaa tat gga ata tat act        1344
Ser Trp Gly Glu Glu Cys Ala Met Lys Gly Lys Tyr Gly Ile Tyr Thr
        435                 440                 445

aag gtt tcc cgg tac gtc aac tgg att aag gaa aaa aca aag cta act        1392
Lys Val Ser Arg Tyr Val Asn Trp Ile Lys Glu Lys Thr Lys Leu Thr
    450                 455                 460

taa                                                                    1395

<210> 28
<211> 464
<212> PRT
<213> Mus musculus

<400> 28

Met Ala Glu Ser Pro Ala Leu Ile Thr Ile Phe Leu Leu Gly Tyr Leu
1               5                   10                  15

Leu Ser Thr Glu Cys Ala Val Phe Leu Asp Arg Glu Asn Ala Thr Lys
            20                  25                  30

Ile Leu Thr Arg Pro Lys Arg Tyr Asn Ser Gly Lys Leu Glu Glu Phe
        35                  40                  45

Val Arg Gly Asn Leu Glu Arg Glu Cys Ile Glu Glu Arg Cys Ser Phe
    50                  55                  60

Glu Glu Ala Arg Glu Val Phe Glu Asn Thr Glu Lys Thr Thr Glu Phe
65                  70                  75                  80

Trp Lys Gln Tyr Val Asp Gly Asp Gln Cys Glu Ser Asn Pro Cys Leu
                85                  90                  95

Asn Gly Gly Ile Cys Lys Asp Asp Ile Ser Ser Tyr Glu Cys Trp Cys
            100                 105                 110

Gln Val Gly Phe Glu Gly Arg Asn Cys Glu Leu Asp Ala Thr Cys Asn
        115                 120                 125

Ile Lys Asn Gly Arg Cys Lys Gln Phe Cys Lys Asn Ser Pro Asp Asn
    130                 135                 140

Lys Val Ile Cys Ser Cys Thr Glu Gly Tyr Gln Leu Ala Glu Asp Gln
145                 150                 155                 160

Lys Ser Cys Glu Pro Thr Val Pro Phe Pro Cys Gly Arg Ala Ser Ile
                165                 170                 175

Ser Tyr Ser Ser Lys Lys Ile Thr Arg Ala Glu Thr Val Phe Ser Asn
            180                 185                 190

Met Asp Tyr Glu Asn Ser Thr Glu Ala Val Phe Ile Gln Asp Asp Ile
        195                 200                 205

Thr Asp Gly Ala Ile Leu Asn Asn Val Thr Glu Ser Ser Glu Ser Leu
    210                 215                 220

Asn Asp Phe Thr Arg Val Val Gly Gly Glu Asn Ala Lys Pro Gly Gln
225                 230                 235                 240

Ile Pro Trp Gln Val Ile Leu Asn Gly Glu Ile Glu Ala Phe Cys Gly
                245                 250                 255

Gly Ala Ile Ile Asn Glu Lys Trp Ile Val Thr Ala Ala His Cys Leu
            260                 265                 270

Lys Pro Gly Asp Lys Ile Glu Val Val Ala Gly Glu Tyr Asn Ile Asp
        275                 280                 285

Lys Lys Glu Asp Thr Glu Gln Arg Arg Asn Val Ile Arg Thr Ile Pro
    290                 295                 300

His His Gln Tyr Asn Ala Thr Ile Asn Lys Tyr Ser His Asp Ile Ala
305                 310                 315                 320

Leu Leu Glu Leu Asp Lys Pro Leu Ile Leu Asn Ser Tyr Val Thr Pro
                325                 330                 335

Ile Cys Val Ala Asn Arg Glu Tyr Thr Asn Ile Phe Leu Lys Phe Gly
            340                 345                 350

Ser Gly Tyr Val Ser Gly Trp Gly Lys Val Phe Asn Lys Gly Arg Gln
        355                 360                 365

Ala Ser Ile Leu Gln Tyr Leu Arg Val Pro Leu Val Asp Arg Ala Thr
    370                 375                 380

Cys Leu Arg Ser Thr Thr Phe Thr Ile Tyr Asn Asn Met Phe Cys Ala
385                 390                 395                 400

Gly Tyr Arg Glu Gly Gly Lys Asp Ser Cys Glu Gly Asp Ser Gly Gly
                405                 410                 415

Pro His Val Thr Glu Val Glu Gly Thr Ser Phe Leu Thr Gly Ile Ile
            420                 425                 430

Ser Trp Gly Glu Glu Cys Ala Met Lys Gly Lys Tyr Gly Ile Tyr Thr
        435                 440                 445

Lys Val Ser Arg Tyr Val Asn Trp Ile Lys Glu Lys Thr Lys Leu Thr
    450                 455                 460

<210>  29
<211>  102
<212>  DNA
<213>  Mus musculus

<220>

<221>  CDS
<222>  (1)..(102)

<400>  29
tgt gaa tca aat cct tgt tta aat ggt gga ata tgc aag gat gat att      48
Cys Glu Ser Asn Pro Cys Leu Asn Gly Gly Ile Cys Lys Asp Asp Ile
1               5                   10                  15

agt tcc tat gaa tgc tgg tgc caa gtt gga ttt gaa gga agg aac tgt      96
Ser Ser Tyr Glu Cys Trp Cys Gln Val Gly Phe Glu Gly Arg Asn Cys
            20                  25                  30

gaa tta                                                              102
Glu Leu

<210>  30
<211>  34
<212>  PRT
<213>  Mus musculus

<400>  30

Cys Glu Ser Asn Pro Cys Leu Asn Gly Gly Ile Cys Lys Asp Asp Ile
1               5                   10                  15

Ser Ser Tyr Glu Cys Trp Cys Gln Val Gly Phe Glu Gly Arg Asn Cys
            20                  25                  30

Glu Leu

<210>  31
<211>  12
<212>  PRT
<213>  Mus musculus

<400>  31

Cys Thr Asp Leu Val Ala Asn Tyr Ser Cys Glu Cys
1               5                   10

<210>  32
<211>  27
<212>  DNA
<213>  Artificial

<220>
<223>  synthetic DNA

<400>  32
tgcaacccga acccctgtga aaatggt                                          27

<210>  33
<211>  25
<212>  DNA
<213>  Artificial

<220>
<223>  synthetic DNA

<400>  33
ttcctcctct gcgcagccca gcagc                                            25

<210>  34
<211>  26
<212>  DNA
<213>  Artificial

<220>
<223>  synthetic DNA

<400>  34
tgtgaagctg agccttgcag aaatgg                                           26

<210>  35
<211>  26
<212>  DNA

<213>  Artificial

<220>
<223>  synthetic DNA

<400>  35
atattgacaa tttcgtccca taaatt                                          26


<210>  36
<211>  34
<212>  DNA
<213>  Artificial

<220>
<223>  synthetic DNA

<400>  36
aaagatcttg tgaatcaaat ccttgtttaa atgg                                 34


<210>  37
<211>  35
<212>  DNA
<213>  Artificial

<220>
<223>  synthetic DNA

<400>  37
ttctcgagtt aattcacagt tccttccttc aaatc                                35